# EUROPEAN PATENT SPECIFICATION

(11) **EP 4 514 769 B1**
(45) Date of publication and mention of the grant of the patent: **15.04.2026**
(21) Application number: 23723183.2
(22) Date of filing: 28.04.2023
(51) Int. Cl.: C07C 69/24, C07C 69/36, C07C 69/38, C07C 69/54, C07C 69/56, C07C 69/606, C07C 69/67, C07C 69/68, C07C 69/708, C07C 69/72, C07C 69/74, C07C 69/96, C11B 9/00

(54) **MUSKY ODORANT**
MOSCHUSRIECHSTOFF
SUBSTANCE ODORANTE À BASE DE MUSC

(30) Priority: 29.04.2022 EP 22170890
(43) Date of publication of application: 05.03.2025
(73) Proprietor: Firmenich SA, 1242 Satigny (CH)
(72) Inventor: LEM, George, 1242 Satigny (CH)
(74) Representative: dsm-firmenich IP
(86) International application number: PCT/EP2023/061213
(87) International publication number: WO 2023/209139

(56) References cited:
- EP-A1- 0 472 966
- EP-A1- 1 262 474
- US-A1- 2006 046 955

## Description

### Technical field

The present invention relates to the field of perfumery. More particularly, it concerns a compound of formula (I) as defined herein below, and its uses as perfuming ingredients. Therefore, following what is mentioned herein, the present invention comprises the invention compound as part of a perfuming composition or of a perfumed consumer product.

### Background of the Invention

Ingredients imparting musk notes are very appreciated and widely used in perfumery as they are considered as one of a key perfumery base notes. Since the 90s and the discovery of Helvetolide^{®} (trademark from Firmenich SA, Suisse) reported in EP0472966, the interest toward this family of alicyclic compounds possessing musk odor allied with fruity and amber notes increased. In said prior art, the 3,3-dimethylcyclopentyl analogues are described as weaker and lacking tenacity. In US2006046955, unsaturated 6-membered ring analogues were reported. However, those compounds, and in particular 2-[1-(3,3-dimethyl-1-cyclohexen-1-yl)ethoxy]-2-methylpropyl propanoate, were less powerful than Helvetolide^{®}.

EP 1 262 474 A1 describes odourant compounds selected from cycloalkane carboxylic esters of 2-[1,(3,3-dimethylcyclohexyl)ethoxy]-2-methylpropan-1-ol.

So there is still a need to develop an alicyclic musk compound imparting a powerful and substantive musky note.

The present invention provides a novel alicyclic perfumery ingredient of formula (I) comprising an unsaturated 5 membered ring, which has never been reported, imparting musk note while maintaining a good tenacity similar to Helvetolide^{®}. The prior art document mentioned above does not disclose the compounds of formula (I) or the organoleptic properties of the compounds of formula (I).

### Description of the invention

Surprisingly, it has now been discovered that a compound of formula (I) possesses a strong musky note particularly appreciated in perfumery.

A first object of the present invention is a compound of formula (I) 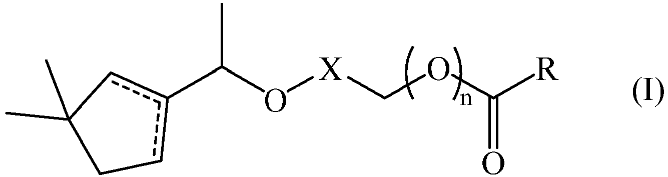 in the form of any one of its stereoisomers or as a mixture thereof, wherein one dotted line represents a carbon-carbon double bond and the other dotted line represents a carbon-carbon single bond; n is 0 or 1; X is a C(=O) or C(Me)₂ group; R is a hydrogen atom, a C₁₋₆ alkyl, C₁₋₆ alkoxyl, C₂₋₆ alkenyl, C₂₋₆ alkynyl or C₃₋₆ cycloalkyl group, a C(=O)R' group or a CH(OR'')R'' group wherein R' is a C₁₋₃ alkyl or C₁₋₃ alkoxyl group and R'', independently from each other, is a hydrogen atom or a C₁₋₃ alkyl group.

Said compound can be used as perfuming ingredient, for instance to impart odor notes of the musky type with red fruit facet.

For the sake of clarity, by the expression "any one of its stereoisomers or a mixture thereof", or the similar, it is meant the normal meaning understood by a person skilled in the art, i.e. that the compound of formula (I) can be a pure enantiomer or diastereomer. In other words, the compound of formula (I) may possess several stereocenters and each of said stereocenter can have two different stereochemistries (e.g. R or S). The compound of formula (I) may even be in the form of a pure enantiomer or in the form of a mixture of enantiomers or diastereoisomers. The compound of formula (I) can be in a racemic form or scalemic form. Therefore, the compound of formula (I) can be one stereoisomers or in the form of a composition of matter comprising, or consisting of, various stereoisomers.

For the sake of clarity, by the expression "wherein one dotted line represents a carbon-carbon double bond and the other dotted line represents a carbon-carbon single bond", or the similar, it is meant the normal meaning understood by a person skilled in the art, i.e. that the whole bonding (solid and dotted line) between the carbon atoms connected by said dotted line is a carbon-carbon single or double bond. In other words, compound of formula (I) comprises a cyclopentenyl group and may be of formula 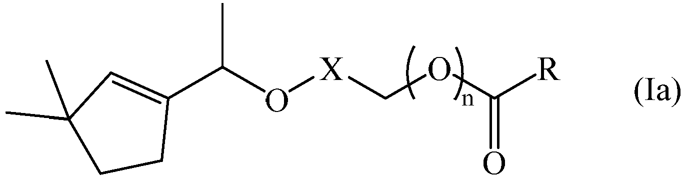 or 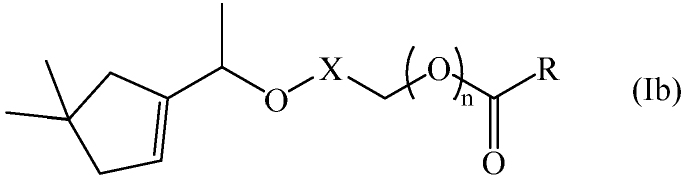 in the form of any one of its stereoisomers or as a mixture thereof, wherein X, n and R have the same meaning as defined above.

According to any embodiments of the invention, the invention compound may be in a form of a composition of matter comprising compounds of formula (Ia) and (Ib). More particularly, the composition of matter may comprise from 20 to 90% w/w of compound of formula (Ia) and from 5 to 50% w/w of compound of formula (Ib), the percentage being relative to the total weight of the composition of matter.

The terms "alkyl", "alkoxyl", "alkenyl" and "alkynyl" are understood as comprising branched and linear alkyl, alkoxyl, alkenyl and alkynyl groups. The term "alkenyl" is understood as comprising 1 olefinic double bond. The terms "alkynyl" is understood as comprising 1 triple bond. The terms "cycloalkyl" is understood as comprising a monocyclicgroup.

According to any one of the above embodiments of the invention, said compounds (I) are C₁₂-C₂₀ compounds, preferably a C₁₄-C₂₇.

According to a particular embodiment, when X is a C(=O), then n is 0.

According to a particular embodiment, when n is 0, then R may be a C₁₋₆ alkoxyl group, particularly, a C₁₋₃ alkoxyl group, particularly, a C₁₋₂ alkoxyl group, even more particularly, an ethoxyl group.

According to any one of the above embodiments of the invention, n may be 1.

According to any one of the above embodiments of the invention, X may be a C(Me)₂ group.

According to any one of the above embodiments of the invention, the compound of formula (I) is a compound of formula 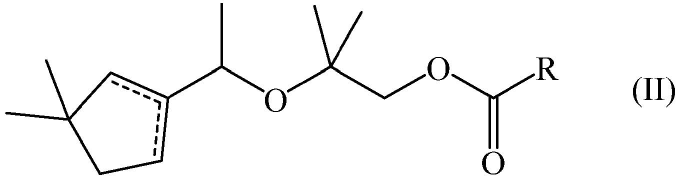 in the form of any one of its stereoisomers or as a mixture thereof, wherein the dotted lines and R have the same meaning as defined above.

According to any one of the above embodiments of the invention, R may be a hydrogen atom, a C₁₋₄ alkyl, C₁₋₄ alkoxyl, C₂₋₄ alkenyl, C₂₋₄ alkynyl or C₃₋₆ cycloalkyl group, a C(=O)R' group or a CH(OR'')R'' group wherein R' is a C₁₋₃ alkyl or C₁₋₃ alkoxyl group and R'', independently from each other, is a hydrogen atom or a C₁₋₃ alkyl group. Particularly, R may be a hydrogen atom, a C₁₋₃ alkyl, C₁₋₃ alkoxyl, C₂₋₃ alkenyl, C₂₋₃ alkynyl or C₃₋₆ cycloalkyl group, a C(=O)R' group or a CH(OR'')R'' group wherein R' is a C₁₋₂ alkyl or C₁₋₂ alkoxyl group and R'', independently from each other, is a hydrogen atom or a C₁₋₂ alkyl group. Particularly, R may be a hydrogen atom, a C₁₋₃ alkyl, C₁₋₃ alkoxyl, C₂₋₃ alkenyl, C₂₋₃ alkynyl or C₃₋₆ cycloalkyl group, a CH(OH)Me group, a CH₂OH group or a C(=O)R' group wherein R' is a C₁₋₂ alkyl or C₁₋₂ alkoxyl group. Particularly, R may be a C₁₋₃ alkyl, C₁₋₃ alkoxyl, C₂₋₃ alkenyl, C₂₋₃ alkynyl or C₃ cycloalkyl group or a C(=O)R' group wherein R' is a C₁₋₂ alkyl group. Particularly, R may be an ethyl, propyl, ethoxy, prop-1-en-1-yl, 2-ethynyl or a cyclopropyl group or a C(=O)Me group. Particularly, R may be an ethyl, ethoxy or prop-1-en-1-yl group. Even more particularly, R may be an ethyl group.

According to any one of the above embodiments of the invention, the compound of formula (I) is a compound of formula 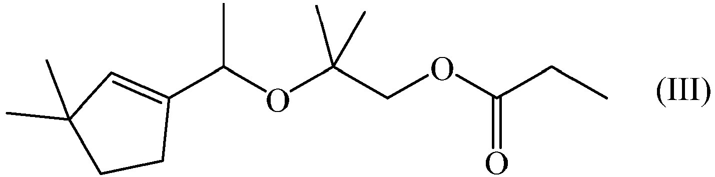 in the form of any one of its stereoisomers or as a mixture thereof.

As specific examples of the invention's compounds, one may cite, as non-limiting example, a composition of matter comprising 2-[1-(3,3-dimethyl-1-cyclopenten-1-yl)ethoxy]-2-methylpropyl propionate and 2-[1-(4,4-dimethyl-1-cyclopenten-1-yl)ethoxy]-2-methylpropyl propionate in a ratio of 80 : 20 imparting a musky note allied with red fruity note, in particular raspberry. Said invention's composition of matter also possesses the cooked apple note of the delta damascene. Said invention's composition of matter possesses a powerful top and heart note while maintaining a high substantivity, comparable to Helvetolide^{®}.

As other example, one may cite 2-[1-(3,3-dimethyl-1-cyclopenten-1-yl)ethoxy]-2-methylpropyl propionate, which possesses an odor similar to the one mentioned above.

As other specific, but non-limiting, examples of the invention's compounds, one may cite the following ones in Table 1.

**Table 1 : Invention's compounds and comparative compounds and their odor properties**

| **Invention's compound structure and name** | **Odor notes** |
|---|---|
| 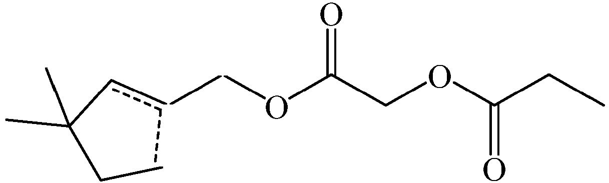 Composition of matter comprising 2-[1-(3,3-dimethyl-1-cyclopenten-1-yl)ethoxy]-2-oxoethyl propionate and 2-[1-(4,4-dimethyl-1-cyclopenten-1-yl)ethoxy]-2-oxoethyl propionate | Musky |
| 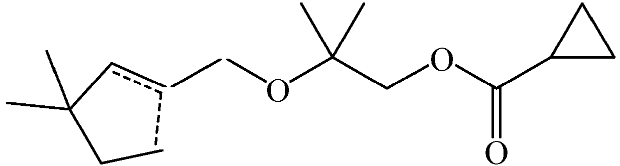 Composition of matter comprising 2-[1-(3,3-dimethyl-1-cyclopenten-1-yl)ethoxy]-2-methylpropyl cyclopropanecarboxylate and 2-[1-(4,4-dimethyl-1-cyclopenten-1-yl)ethoxy]-2-methylpropyl cyclopropanecarboxylate | Musky |
| 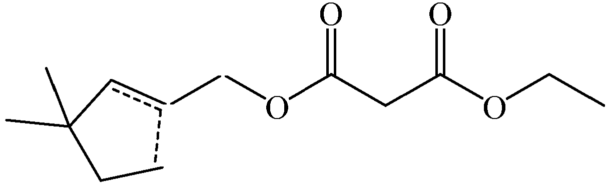 Composition of matter comprising 1-(3,3-dimethyl-1-cyclopenten-1-yl)ethyl ethyl malonate and 1-(4,4-dimethyl-1-cyclopenten-1-yl)ethyl ethyl malonate | Musky |
| 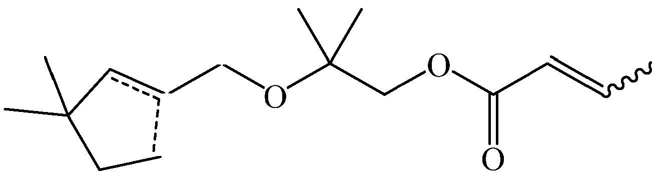 Composition of matter comprising 2-[1-(3,3-dimethyl-1-cyclopenten-1-yl)ethoxy]-2-methylpropyl 2-butenoate and 2-[1-(4,4-dimethyl-1-cyclopenten-1-yl)ethoxy]-2-methylpropyl 2-butenoate | Musky |
| 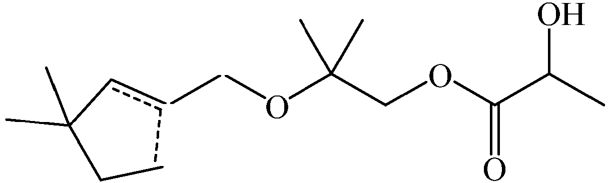 Composition of matter comprising 2-[1-(3,3-dimethyl-1-cyclopenten-1-yl)ethoxy]-2-methylpropyl 2-hydroxypropanoate and 2-[1-(4,4-dimethyl-1-cyclopenten-1-yl)ethoxy]-2-methylpropyl 2-hydroxypropanoate | Musky |
| 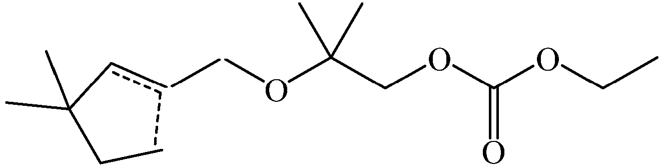 Composition of matter comprising 2-[1-(3,3-dimethyl-1-cyclopenten-1-yl)ethoxy]-2-methylpropyl ethyl carbonate and 2-[1-(4,4-dimethyl-1-cyclopenten-1-yl)ethoxy]-2-methylpropyl ethyl carbonate | Musky |
| 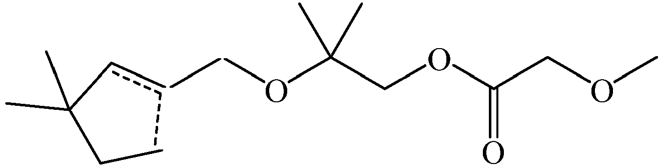 Composition of matter comprising 2-[1-(3,3-dimethyl-1-cyclopenten-1-yl)ethoxy]-2-methylpropyl methoxyacetate and 2-[1-(4,4-dimethyl-1-cyclopenten-1-yl)ethoxy]-2-methylpropyl methoxyacetate | Musky |
| 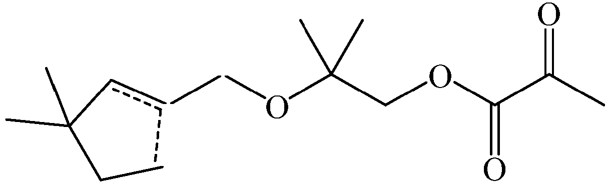 Composition of matter comprising 2-[1-(3,3-dimethyl-1-cyclopenten-1-yl)ethoxy]-2-methylpropyl 2-oxopropanoate and 2-[1-(4,4-dimethyl-1-cyclopenten-1-yl)ethoxy]-2-methylpropyl 2-oxopropanoate | Musky |
| 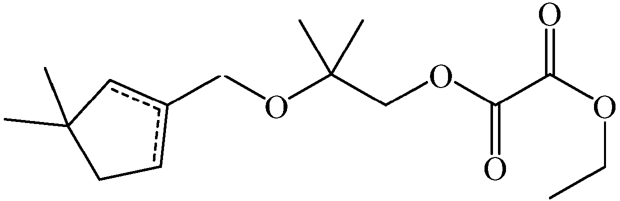 Composition of matter comprising 2-[1-(3,3-dimethyl-1-cyclopenten-1-yl)ethoxy]-2-methylpropyl ethyl oxalate and 2-[1-(4,4-dimethyl-1-cyclopenten-1-yl)ethoxy]-2-methylpropyl ethyl oxalate | Musky |
| 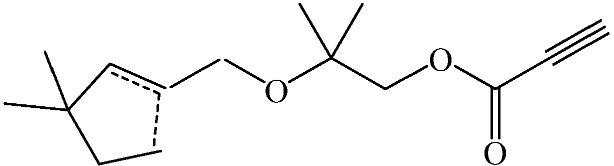 Composition of matter comprising 2-[1-(3,3-dimethyl-1-cyclopenten-1-yl)ethoxy]-2-methylpropyl propiolate and 2-[1-(4,4-dimethyl-1-cyclopenten-1-yl)ethoxy]-2-methylpropyl propiolate | Musky |
| 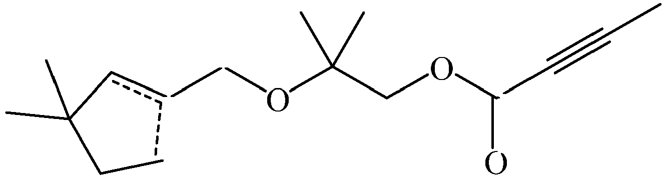 Composition of matter comprising 2-[1-(3,3-dimethyl-1-cyclopenten-1-yl)ethoxy]-2-methylpropyl 2-butynoate and 2-[1-(4,4-dimethyl-1-cyclopenten-1-yl)ethoxy]-2-methylpropyl 2-butynoate | Musky |
| 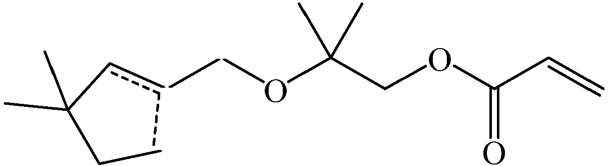 | Musky |
| Composition of matter comprising 2-[1-(3,3-dimethyl-1-cyclopenten-1-yl)ethoxy]-2-methylpropyl acrylate and 2-[1-(4,4-dimethyl-1-cyclopenten-1-yl)ethoxy]-2-methylpropyl acrylate | |
| 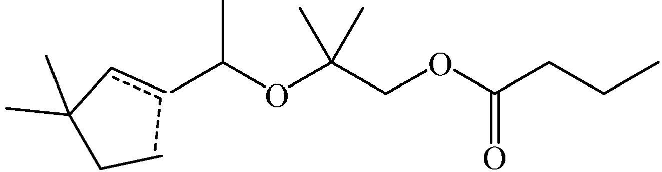 Composition of matter comprising 2-[1-(3,3-dimethyl-1-cyclopenten-1-yl)ethoxy]-2-methylpropyl butyrate and 2-[1-(4,4-dimethyl-1-cyclopenten-1-yl)ethoxy]-2-methylpropyl butyrate | Musky |
| | |

| **Comparative compounds structure and name** | **Odor notes** |
|---|---|
| 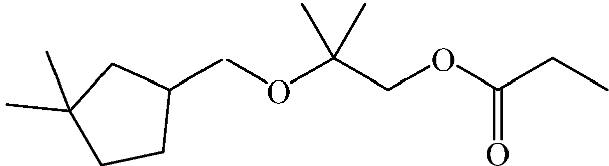 2-(1-(3,3-dimethylcyclopentyl)ethoxy)-2-methylpropyl propionate | Musky weak |
| 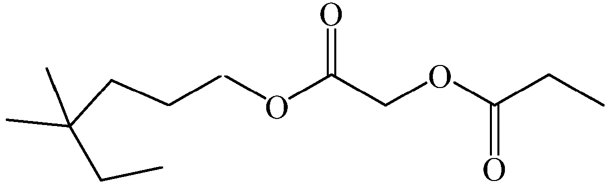 2-(1-(3,3-dimethylcyclopentyl)ethoxy)-2-oxoethyl propionate | Very weak |

For the sake of clarity, by the wavy bond in compound of the above table; i.e. the composition of matter comprising 2-[1-(3,3-dimethyl-1-cyclopenten-1-yl)ethoxy]-2-methylpropyl 2-butenoate and 2-[1-(4,4-dimethyl-1-cyclopenten-1-yl)ethoxy]-2-methylpropyl 2-butenoate, or the similar, it is meant the normal meaning understood by a person skilled in the art, i.e. that the double bond may have a cis configuration corresponding to the Z isomer, a trans configuration corresponding to the E isomer or a mixture thereof. In other words, said compound may be in the form of its E or Z isomer or of a mixture thereof, e.g. the invention process leads to a composition of matter consisting of one or more of said compounds, having the same chemical structure but differing by the configuration of the double bond.

The invention's compound is surprisingly very powerful. All of the comparative compounds cited below, being structurally close to the invention compounds, impart a less interesting and weaker odor note. Only the invention's compounds impart a powerful and substantive note.

According to a particular embodiment of the invention, the compounds of formula (I) are 2-[1-(3,3-dimethyl-1-cyclopenten-1-yl)ethoxy]-2-methylpropyl propionate, 2-[1-(4,4-dimethyl-1-cyclopenten-1-yl)ethoxy]-2-methylpropyl propionate or a mixture thereof; 2-[1-(3,3-dimethyl-1-cyclopenten-1-yl)ethoxy]-2-oxoethyl propionate, 2-[1-(4,4-dimethyl-1-cyclopenten-1-yl)ethoxy]-2-oxoethyl propionate or a mixture thereof; 2-[1-(3,3-dimethyl-1-cyclopenten-1-yl)ethoxy]-2-methylpropyl cyclopropanecarboxylate, 2-[1-(4,4-dimethyl-1-cyclopenten-1-yl)ethoxy]-2-methylpropyl cyclopropanecarboxylate or a mixture thereof; 1-(3,3-dimethyl-1-cyclopenten-1-yl)ethyl ethyl malonate, 1-(4,4-dimethyl-1-cyclopenten-1-yl)ethyl ethyl malonate or a mixture thereof; 2-[1-(3,3-dimethyl-1-cyclopenten-1-yl)ethoxy]-2-methylpropyl 2-butenoate, 2-[1-(4,4-dimethyl-1-cyclopenten-1-yl)ethoxy]-2-methylpropyl 2-butenoate or a mixture thereof; 2-[1-(3,3-dimethyl-1-cyclopenten-1-yl)ethoxy]-2-methylpropyl 2-hydroxypropanoate, 2-[1-(4,4-dimethyl-1-cyclopenten-1-yl)ethoxy]-2-methylpropyl 2-hydroxypropanoate or a mixture thereof; 2-[1-(3,3-dimethyl-1-cyclopenten-1-yl)ethoxy]-2-methylpropyl ethyl carbonate, 2-[1-(4,4-dimethyl-1-cyclopenten-1-yl)ethoxy]-2-methylpropyl ethyl carbonate or a mixture thereof; 2-[1-(3,3-dimethyl-1-cyclopenten-1-yl)ethoxy]-2-methylpropyl methoxyacetate, 2-[1-(4,4-dimethyl-1-cyclopenten-1-yl)ethoxy]-2-methylpropyl methoxyacetate or a mixture thereof; 2-[1-(3,3-dimethyl-1-cyclopenten-1-yl)ethoxy]-2-methylpropyl 2-oxopropanoate, 2-[1-(4,4-dimethyl-1-cyclopenten-1-yl)ethoxy]-2-methylpropyl 2-oxopropanoate or a mixture thereof; 2-[1-(3,3-dimethyl-1-cyclopenten-1-yl)ethoxy]-2-methylpropyl ethyl oxalate, 2-[1-(4,4-dimethyl-1-cyclopenten-1-yl)ethoxy]-2-methylpropyl ethyl oxalate or a mixture thereof; 2-[1-(3,3-dimethyl-1-cyclopenten-1-yl)ethoxy]-2-methylpropyl propiolate, 2-[1-(4,4-dimethyl-1-cyclopenten-1-yl)ethoxy]-2-methylpropyl propiolate or a mixture thereof; 2-[1-(3,3-dimethyl-1-cyclopenten-1-yl)ethoxy]-2-methylpropyl 2-butynoate, 2-[1-(4,4-dimethyl-1-cyclopenten-1-yl)ethoxy]-2-methylpropyl 2-butynoate or a mixture thereof; 2-[1-(3,3-dimethyl-1-cyclopenten-1-yl)ethoxy]-2-methylpropyl acrylate, 2-[1-(4,4-dimethyl-1-cyclopenten-1-yl)ethoxy]-2-methylpropyl acrylate or a mixture thereof; 2-[1-(3,3-dimethyl-1-cyclopenten-1-yl)ethoxy]-2-methylpropyl butyrate, 2-[1-(4,4-dimethyl-1-cyclopenten-1-yl)ethoxy]-2-methylpropyl butyrate or a mixture thereof. Particularly, the compounds of formula (I) may be selected from the group consisting of 2-[1-(3,3-dimethyl-1-cyclopenten-1-yl)ethoxy]-2-methylpropyl propionate, 2-[1-(4,4-dimethyl-1-cyclopenten-1-yl)ethoxy]-2-methylpropyl propionate or a mixture thereof; 2-[1-(3,3-dimethyl-1-cyclopenten-1-yl)ethoxy]-2-methylpropyl cyclopropanecarboxylate, 2-[1-(4,4-dimethyl-1-cyclopenten-1-yl)ethoxy]-2-methylpropyl cyclopropanecarboxylate or a mixture thereof; 1-(3,3-dimethyl-1-cyclopenten-1-yl)ethyl ethyl malonate, 1-(4,4-dimethyl-1-cyclopenten-1-yl)ethyl ethyl malonate or a mixture thereof; 2-[1-(3,3-dimethyl-1-cyclopenten-1-yl)ethoxy]-2-methylpropyl 2-butenoate, 2-[1-(4,4-dimethyl-1-cyclopenten-1-yl)ethoxy]-2-methylpropyl 2-butenoate or a mixture thereof; 2-[1-(3,3-dimethyl-1-cyclopenten-1-yl)ethoxy]-2-methylpropyl ethyl carbonate, 2-[1-(4,4-dimethyl-1-cyclopenten-1-yl)ethoxy]-2-methylpropyl ethyl carbonate or a mixture thereof; 2-[1-(3,3-dimethyl-1-cyclopenten-1-yl)ethoxy]-2-methylpropyl 2-oxopropanoate, 2-[1-(4,4-dimethyl-1-cyclopenten-1-yl)ethoxy]-2-methylpropyl 2-oxopropanoate or a mixture thereof; 2-[1-(3,3-dimethyl-1-cyclopenten-1-yl)ethoxy]-2-methylpropyl propiolate, 2-[1-(4,4-dimethyl-1-cyclopenten-1-yl)ethoxy]-2-methylpropyl propiolate or a mixture thereof; 2-[1-(3,3-dimethyl-1-cyclopenten-1-yl)ethoxy]-2-methylpropyl butyrate, 2-[1-(4,4-dimethyl-1-cyclopenten-1-yl)ethoxy]-2-methylpropyl butyrate or a mixture thereof. Even more particularly, the compound of formula (I) is a composition of matter comprising 2-[1-(3,3-dimethyl-1-cyclopenten-1-yl)ethoxy]-2-methylpropyl propionate and 2-[1-(4,4-dimethyl-1-cyclopenten-1-yl)ethoxy]-2-methylpropyl propionate.

When the odor of the invention's compounds is compared with that of the prior art compound 2-[1-(3,3-dimethyl-1-cyclohexen-1-yl)ethoxy]-2-methylpropyl propanoate, then the invention's compounds distinguish themselves by a clearly stronger musk-like note allied with raspberry note and by lacking the powdery-like note so characteristic of the prior art compound(s). Moreover, the invention's compound imparts a clearly stronger and more substantive note being similar to Helvetolide^{®}.

The odor of the invention's compounds distinguished also from the prior art compounds by lacking, or not possessing significant, animalic and ambrette notes, which are characteristic of the prior art compounds. Said differences lend the invention's compounds and the prior art compounds to be each suitable for different uses, i.e. to impart different organoleptic impressions.

As mentioned above, the invention concerns the use of a compound of formula (I) as a perfuming ingredient. In other words, it concerns a method or a process to confer, enhance, improve or modify the odor properties of a perfuming composition or of a perfumed article or of a surface, which method comprises adding to said composition or article an effective amount of at least a compound of formula (I), e.g. to impart its typical note. Understood that the final hedonic effect may depend on the precise dosage and on the organoleptic properties of the invention's compound, but anyway the addition of the invention's compound will impart to the final product its typical touch in the form of a note, touch or aspect depending on the dosage.

By "use of a compound of formula (I)" it has to be understood here also the use of any composition containing a compound of formula (I) and which can be advantageously employed in the perfumery industry.

Said compositions, which in fact can be advantageously employed as perfuming ingredients, are also an object of the present invention.

Therefore, another object of the present invention is a perfuming composition comprising:
i) as a perfuming ingredient, at least one invention's compound as defined above;
ii) at least one ingredient selected from the group consisting of a perfumery carrier and a perfumery base; and
iii) optionally at least one perfumery adjuvant.

By "perfumery carrier" it is meant here a material which is practically neutral from a perfumery point of view, i.e. that does not significantly alter the organoleptic properties of perfuming ingredients. Said carrier may be a liquid or a solid.

As liquid carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples, solvents such as butylene or propylene glycol, glycerol, dipropyleneglycol and its monoether, 1,2,3-propanetriyl triacetate, dimethyl glutarate, dimethyl adipate 1,3-diacetyloxypropan-2-yl acetate, diethyl phthalate, isopropyl myristate, Abalyn^{®} (rosin resins, available from Eastman), benzyl benzoate, benzyl alcohol, 2-(2-ethoxyethoxy)-1-ethanol, tri-ethyl citrate or mixtures thereof, which are the most commonly used or also naturally derived solvents like glycerol or various vegetable oils such as palm oil, sunflower oil or linseed oil. For the compositions which comprise both a perfumery carrier and a perfumery base, other suitable perfumery carriers than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company), or hydrogenated castors oils such as those known under the trademark Cremophor^{®} RH 40 (origin: BASF).

Solid carrier is meant to designate a material to which the perfuming composition or some element of the perfuming composition can be chemically or physically bound. In general such solid carriers are employed either to stabilize the composition, or to control the rate of evaporation of the compositions or of some ingredients. Solid carriers are of current use in the art and a person skilled in the art knows how to reach the desired effect. However by way of non-limiting examples of solid carriers, one may cite absorbing gums or polymers or inorganic materials, such as porous polymers, cyclodextrines, dextrines, maltodextrines wood based materials, organic or inorganic gels, clays, gypsum talc or zeolites.

As other non-limiting examples of solid carriers, one may cite encapsulating materials. Examples of such materials may comprise wall-forming and plasticizing materials, such as glucose syrups, natural or modified starches, hydrocolloids, cellulose derivatives, polyvinyl acetates, polyvinylalcohols, proteins or pectins, plant gums such as acacia gum (Gum Arabic), urea, sodium chloride, sodium sulphate, zeolite, sodium carbonate, sodium bicarbonate, clay, talc, calcium carbonate, magnesium sulfate, gypsum, calcium sulfate, magnesium oxide, zinc oxide, titanium dioxide, calcium chloride, potassium chloride, magnesium chloride, zinc chloride, carbohydrates, saccharides such as sucrose, mono-, di-, and polysaccharides and derivatives such as chitosan, starch, cellulose, carboxymethyl methylcellulose, methylcellulose, hydroxyethyl cellulose, ethyl cellulose, propyl cellulose, polyols/sugar alcohols such as sorbitol, maltitol, xylitol, erythritol, and isomalt, polyethylene glycol (PEG), polyvinyl pyrrolidin (PVP), polyvinyl alcohol, acrylamides, acrylates, polyacrylic acid and related, maleic anhydride copolymers, aminefunctional polymers, vinyl ethers, styrenes, polystyrenesulfonates, vinyl acids, ethylene glycol-propylene glycol block copolymers, vegetable gums, gum acacia, pectins, xanthanes, alginates, carragenans, citric acid or any water soluble solid acid, fatty alcohols or fatty acids and mixtures thereof, or yet the materials cited in reference texts such as H. Scherz, Hydrokolloide: Stabilisatoren, Dickungs- und Geliermittel in Lebensmitteln, Band 2 der Schriftenreihe Lebensmittelchemie, Lebensmittelqualität, Behr's Verlag GmbH & Co., Hamburg, 1996. The encapsulation is a well-known process to a person skilled in the art, and may be performed, for instance, by using techniques such as spray-drying, agglomeration or yet extrusion; or consists of a coating encapsulation, including coacervation and complex coacervation techniques.

As non-limiting examples of solid carriers, one may cite in particular the core-shell capsules with resins of aminoplast, polyamide, polyester, polyurea or polyurethane type or a mixture threof (all of said resins are well known to a person skilled in the art) using techniques like phase separation process induced by polymerization, interfacial polymerization, coacervation or altogether (all of said techniques have been described in the prior art), optionally in the presence of a polymeric stabilizer or of a cationic copolymer.

Resins may be produced by the polycondensation of an aldehyde (e.g. formaldehyde, 2,2-dimethoxyethanal, glyoxal, glyoxylic acid or glycolaldehyde and mixtures thereof) with an amine such as urea, benzoguanamine, glycouryl, melamine, methylol melamine, methylated methylol melamine, guanazole and the like, as well as mixtures thereof. Alternatively one may use preformed resins alkylolated polyamines such as those commercially available under the trademark Urac^{®} (origin: Cytec Technology Corp.), Cymel^{®} (origin: Cytec Technology Corp.), Urecoll^{®} or Luracoll^{®} (origin: BASF).

Other resins are the ones produced by the polycondensation of a polyol, like glycerol, and a polyisocyanate, like a trimer of hexamethylene diisocyanate, a trimer of isophorone diisocyanate or xylylene diisocyanate or a Biuret of hexamethylene diisocyanate or a trimer of xylylene diisocyanate with trimethylolpropane (known with the tradename of Takenate^{®}, origin: Mitsui Chemicals), among which a trimer of xylylene diisocyanate with trimethylolpropane and a Biuret of hexamethylene diisocyanate are preferred.

Some of the seminal literature related to the encapsulation of perfumes by polycondensation of amino resins, namely melamine based resins with aldehydes includes articles such as those published by K. Dietrich et al. Acta Polymerica, 1989, vol. 40, pages 243, 325 and 683, as well as 1990, Vol. 41, page 91. Such articles already describe the various parameters affecting the preparation of such core-shell microcapsules following prior art methods that are also further detailed and exemplified in the patent literature. US 4'396'670, to the Wiggins Teape Group Limited is a pertinent early example of the latter. Since then, many other authors have enriched the literature in this field and it would be impossible to cover all published developments here, but the general knowledge in encapsulation technology is very significant. More recent publications of pertinence, which disclose suitable uses of such microcapsules, are represented for example by the article of K. Bruyninckx and M. Dusselier, ACS Sustainable Chemistry & Engineering, 2019, vol. 7, pages 8041-8054.

By "perfumery base" what is meant here is a composition comprising at least one perfuming co-ingredient.

Said perfuming co-ingredient is not of formula (I). Moreover, by "perfuming co-ingredient" it is meant here a compound, which is used in a perfuming preparation or a composition to impart a hedonic effect; i.e. used for the primary purpose of conferring or modulating an odor. In other words such a co-ingredient, to be considered as being a perfuming one, must be recognized by a person skilled in the art as being able to impart or modify in a positive or pleasant way the odor of a composition, and not just as having an odor. The perfuming ingredient may impart an additional benefit beyond that of modifying or imparting an odor, such as long-lasting, blooming, malodour counteraction, antimicrobial effect, antiviral effect, microbial stability, or pest control.

The nature and type of the perfuming co-ingredients present in the base do not warrant a more detailed description here, which in any case would not be exhaustive, the skilled person being able to select them on the basis of his general knowledge and according to the intended use or application and the desired organoleptic effect. In general terms, these perfuming co-ingredients belong to chemical classes as varied as alcohols, lactones, aldehydes, ketones, esters, ethers, acetates, nitriles, terpenoids, nitrogenous or sulphurous heterocyclic compounds and essential oils, and said perfuming co-ingredients can be of natural or synthetic origin.

In particular one may cite perfuming co-ingredients which are commonly used in perfume formulations, such as:
- Aldehydic ingredients: decanal, dodecanal, 2-methyl-undecanal, 10-undecenal, octanal, nonanal and/or nonenal;
- Aromatic-herbal ingredients: eucalyptus oil, camphor, eucalyptol, 5-methyltricyclo[6.2.1.0^{2,7}]undecan-4-one, 1-methoxy-3-hexanethiol, 2-ethyl-4,4-dimethyl-1,3-oxathiane, 2,2,7/8,9/10-Tetramethylspiro[5.5]undec-8-en-1-one, menthol and/or alpha-pinene;
- Balsamic ingredients: coumarin, ethylvanillin and/or vanillin;
- Citrus ingredients: dihydromyrcenol, citral, orange oil, linalyl acetate, citronellyl nitrile, orange terpenes, limonene, 1-p-menthen-8-yl acetate and/or 1,4(8)-p-menthadiene;
- Floral ingredients: methyl dihydrojasmonate, linalool, citronellol, phenylethanol, 3-(4-tert-butylphenyl)-2-methylpropanal, hexylcinnamic aldehyde, benzyl acetate, benzyl salicylate, tetrahydro-2-isobutyl-4-methyl-4(2H)-pyranol, beta ionone, methyl 2-(methylamino)benzoate, (E)-3-methyl-4-(2,6,6-trimethyl-2-cyclohexen-1-yl)-3-buten-2-one, (1E)-1-(2,6,6-trimethyl-2-cyclohexen-1-yl)-1-penten-3-one, 1-(2,6,6-trimethyl-1,3-cyclohexadien-1-yl)-2-buten-1-one, (2E)-1-(2,6,6-trimethyl-2-cyclohexen-1-yl)-2-buten-1-one, (2E)-1-[2,6,6-trimethyl-3-cyclohexen-1-yl]-2-buten-1-one, (2E)-1-(2,6,6-trimethyl-1-cyclohexen-1-yl)-2-buten-1-one, 2,5-dimethyl-2-indanmethanol, 2,6,6-trimethyl-3-cyclohexene-1-carboxylate, 3-(4,4-dimethyl-1-cyclohexen-1-yl)propanal, 3-(3,3/1,1-dimethyl-5-indanyl)propanal, hexyl salicylate, 3,7-dimethyl-1,6-nonadien-3-ol, 3-(4-isopropylphenyl)-2-methylpropanal, verdyl acetate, geraniol, p-menth-1-en-8-ol, 4-(1,1-dimethylethyl)-1-cyclohexyle acetate, 1,1-dimethyl-2-phenylethyl acetate, 4-cyclohexyl-2-methyl-2-butanol, amyl salicylate , high cis methyl dihydrojasmonate, 3-methyl-5-phenyl-1-pentanol, verdyl proprionate, geranyl acetate, tetrahydro linalool, cis-7-p-menthanol, propyl (S)-2-(1,1-dimethylpropoxy)propanoate, 2-methoxynaphthalene, 2,2,2-trichloro-1-phenylethyl acetate, 4/3-(4-hydroxy-4-methylpentyl)-3-cyclohexene-1-carbaldehyde, amylcinnamic aldehyde, 8-decen-5-olide, 4-phenyl-2-butanone, isononyle acetate, 4-(1,1-dimethylethyl)-1-cyclohexyl acetate, verdyl isobutyrate and/or mixture of methylionones isomers;
- Fruity ingredients: gamma-undecalactone, 2,2,5-trimethyl-5-pentylcyclopentanone, 2-methyl-4-propyl-1,3-oxathiane, 4-decanolide, ethyl 2-methyl-pentanoate, hexyl acetate, ethyl 2-methylbutanoate, gamma-nonalactone, allyl heptanoate, 2-phenoxyethyl isobutyrate, ethyl 2-methyl-1,3-dioxolane-2-acetate, diethyl 1,4-cyclohexanedicarboxylate, 3-methyl-2-hexen-1-yl acetate, 1-[3,3-dimethylcyclohexyl]ethyl [3-ethyl-2-oxiranyl]acetate and/or diethyl 1,4-cyclohexane dicarboxylate;
- Green ingredients: 2-methyl-3-hexanone (E)-oxime, 2,4-dimethyl-3-cyclohexene-1-carbaldehyde, 2-tert-butyl-1-cyclohexyl acetate, styrallyl acetate, allyl (2-methylbutoxy)acetate, 4-methyl-3-decen-5-ol, diphenyl ether, (Z)-3-hexen-1-ol and/or 1-(5,5-dimethyl-1-cyclohexen-1-yl)-4-penten-1-one;
- Musk ingredients: 1,4-dioxa-5,17-cycloheptadecanedione, (Z)-4-cyclopentadecen-1-one, 3-methylcyclopentadecanone, 1-oxa-12-cyclohexadecen-2-one, 1-oxa-13-cyclohexadecen-2-one, (9Z)-9-cycloheptadecen-1-one, 2-{(1S)-1-[(1R)-3,3-dimethylcyclohexyl]ethoxy}-2-oxoethyl propionate, 3-methyl-5-cyclopentadecen-1-one, 4,6,6,7,8,8-hexamethyl-1,3,4,6,7,8-hexahydrocyclopenta[g]isochromene, (1S,1'R)-2-[1-(3',3'-dimethyl-1'-cyclohexyl)ethoxy]-2-methylpropyl propanoate, oxacyclohexadecan-2-oneand/or (1S,1'R)-[1-(3',3'-dimethyl-1'-cyclohexyl)ethoxycarbonyl]methyl propanoate;
- Woody ingredients: 1-[(1RS,6SR)-2,2,6-trimethylcyclohexyl]-3-hexanol, 3,3-dimethyl-5-[(1R)-2,2,3-trimethyl-3-cyclopenten-1-yl]-4-penten-2-ol, 3,4'-dimethylspiro[oxirane-2,9'-tricyclo[6.2.1.0^{2,7}]undec[4]ene, (1-ethoxyethoxy)cyclododecane, 2,2,9,11-tetramethylspiro[5.5]undec-8-en-1-yl acetate, 1-(octahydro-2,3,8,8-tetramethyl-2-naphtalenyl)-1-ethanone, patchouli oil, terpenes fractions of patchouli oil, Clearwood^{®} (Origin: Firmenich SA), (1'R,E)-2-ethyl-4-(2',2',3'-trimethyl-3'-cyclopenten-1'-yl)-2-buten-1-ol, 2-ethyl-4-(2,2,3-trimethyl-3-cyclopenten-1-yl)-2-buten-1-ol, methyl cedryl ketone, 5-(2,2,3-trimethyl-3-cyclopentenyl)-3-methylpentan-2-ol, 1-(2,3,8,8-tetramethyl-1,2,3,4,6,7,8,8a-octahydronaphthalen-2-yl)ethan-1-one and/or isobornyl acetate;
- Other ingredients (e.g. amber, powdery spicy or watery): dodecahydro-3a,6,6,9a-tetramethyl-naphtho[2,1-b]furan and any of its stereoisomers, heliotropin, anisic aldehyde, eugenol, cinnamic aldehyde, clove oil, 3-(1,3-benzodioxol-5-yl)-2-methylpropanal, 7-methyl-2H-1,5-benzodioxepin-3(4H)-one, 2,5,5-trimethyl-1,2,3,4,4a,5,6,7-octahydro-2-naphthalenol, 1-phenylvinyl acetate, 6-methyl-7-oxa-1-thia-4-azaspiro[4.4]nonane and/or 3-(3-isopropyl-1-phenyl)butanal.

A perfumery base according to the invention may not be limited to the above mentioned perfuming co-ingredients, and many other of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds also known as properfume or profragrance. Non-limiting examples of suitable properfume may include 4-(dodecylthio)-4-(2,6,6-trimethyl-2-cyclohexen-1-yl)-2-butanone, 4-(dodecylthio)-4-(2,6,6-trimethyl-1-cyclohexen-1-yl)-2-butanone, trans-3-(dodecylthio)-1-(2,6,6-trimethyl-3-cyclohexen-1-yl)-1-butanone, 3-(dodecylsulfonyl)-1-(2,6,6-trimethylcyclohex-3-en-1-yl)butan-1-one, a linear polysiloxane co-polymer of (3-mercaptopropyl)(methyl)dimethoxysilane, 2-(dodecylthio)octan-4-one, 2-(dodecylsulfonyl)octan-4-one, 4-oxooctan-2-yl dodecanoate, 2-phenylethyl oxo(phenyl)acetate, 3,7-dimethylocta-2,6-dien-1-yl oxo(phenyl)acetate, (Z)-hex-3-en-1-yl oxo(phenyl)acetate, 3,7-dimethyl-2,6-octadien-1-yl hexadecanoate, bis(3,7-dimethylocta-2,6-dien-1-yl) succinate, (2E,6Z)-2,6-nonadienyl hexadecanoate, (2E,6Z)-2,6-nonadien-1-yl tetradecanoate, (2E,6Z)-2,6-nonadien-1-yl dodecanoate, (2-((2-methylundec-1-en-1-yl)oxy)ethyl)benzene, 1-methoxy-4-(3-methyl-4-phenethoxybut-3-en-1-yl)benzene, (3-methyl-4-phenethoxybut-3-en-1-yl)benzene, 1-(((Z)-hex-3-en-1-yl)oxy)-2-methylundec-1-ene, (2-((2-methylundec-1-en-1-yl)oxy)ethoxy)benzene, 2-methyl-1-(octan-3-yloxy)undec-1-ene, 1-methoxy-4-(1-phenethoxyprop-1-en-2-yl)benzene, 1-methyl-4-(1-phenethoxyprop-1-en-2-yl)benzene, 2-(1-phenethoxyprop-1-en-2-yl)naphthalene, (2-phenethoxyvinyl)benzene, 2-(1-((3,7-dimethyloct-6-en-1-yl)oxy)prop-1-en-2-yl)naphthalene, (2-((2-pentylcyclopentylidene)methoxy)ethyl)benzene, 4-allyl-2-methoxy-1-((2-methoxy-2-phenylvinyl)oxy)benzene, (2-((2-heptylcyclopentylidene)methoxy)ethyl)benzene, 1-methoxy-4-(1-phenethoxyprop-1-en-2-yl)benzene, (2-((2-methyl-4-(2,6,6-trimethylcyclohex-1-en-1-yl)but-1-en-1-yl)oxy)ethyl)benzene, 1-methoxy-4-(2-methyl-3-phenethoxyallyl)benzene, (2-((2-isopropyl-5-methylcyclohexylidene)methoxy)ethyl)benzene, 1-isopropyl-4-methyl-2-((2-pentylcyclopentylidene)methoxy)benzene, 2-methoxy-1-((2-pentylcyclopentylidene)methoxy)-4-propylbenzene, 2-ethoxy-1-((2-methoxy-2-phenylvinyl)oxy)-4-methylbenzene, 3-methoxy-4-((2-methoxy-2-phenylvinyl)oxy)benzaldehyde, 1-isopropyl-2-((2-methoxy-2-phenylvinyl)oxy)-4-methylbenzene, 4-((2-(hexyloxy)-2-phenylvinyl)oxy)-3-methoxybenzaldehyde or a mixture thereof.

By "perfumery adjuvant", it is meant here an ingredient capable of imparting additional added benefit such as a color, a particular light resistance, chemical stability, etc. A detailed description of the nature and type of adjuvant commonly used in perfuming composition cannot be exhaustive, but it has to be mentioned that said ingredients are well known to a person skilled in the art. One may cite as specific non-limiting examples the following: viscosity agents (e.g. surfactants, thickeners, gelling and/or rheology modifiers), stabilizing agents (e.g. preservatives, antioxidant, heat/light and or buffers or chelating agents, such as BHT), coloring agents (e.g. dyes and/or pigments), preservatives (e.g. antibacterial or antimicrobial or antifungal or anti irritant agents), abrasives, skin cooling agents, fixatives, insect repellants, ointments, vitamins and mixtures thereof. By "fixative" also called "modulator", it is understood here an agent having the capacity to affect the manner in which the odour, and in particular the evaporation rate and intensity, of the compositions incorporating said modulator can be perceived by an observer or user thereof, over time, as compared to the same perception in the absence of the modulator. In particular, the modulator allows prolonging the time during which their fragrance is perceived. Non-limiting examples of suitable modulators may include methyl glucoside polyol; ethyl glucoside polyol; propyl glucoside polyol; isocetyl alcohol; PPG-3 myristyl ether; neopentyl glycol diethylhexanoate; sucrose laurate; sucrose dilaurate, sucrose myristate, sucrose palmitate, sucrose stearate, sucrose distearate, sucrose tristearate, hyaluronic acid disaccharide sodium salt, sodium hyaluronate, propylene glycol propyl ether; dicetyl ether; polyglycerin-4 ethers; isoceteth-5; isoceteth-7, isoceteth-10; isoceteth-12; isoceteth-15; isoceteth-20; isoceteth-25; isoceteth-30; disodium lauroamphodipropionate; hexaethylene glycol monododecyl ether; and their mixtures; neopentyl glycol diisononanoate; cetearyl ethylhexanoate; panthenol ethyl ether, DL-panthenol, N-hexadecyl n-nonanoate, noctadecyl n-nonanoate, a profragrance, cyclodextrin, an encapsulation, and a combination thereof. At most 20% by weight, based on the total weight of the perfuming composition, of the modulator may be incorporated into the perfumed consumer product.

It is understood that a person skilled in the art is perfectly able to design optimal formulations for the desired effect by admixing the above mentioned components of a perfuming composition, simply by applying the standard knowledge of the art as well as by trial and error methodologies.

An invention's composition consisting of at least one compound of formula (I) and at least one perfumery carrier consists of a particular embodiment of the invention as well as a perfuming composition comprising at least one compound of formula (I), at least one perfumery carrier, at least one perfumery base, and optionally at least one perfumery adjuvant.

According to a particular embodiment, the perfuming composition is in the form of a microcapsule, preferably a microcapsule slurry, comprising at least one compound of formula (I). In one embodiment, the at least one compound of formula (I) is encapsulated in a core-shell microcapsule wherein the at least one compound of formula (I) is contained in the core surrounded by the shell. In one embodiment, the shell of the microcapsule protects the compound of formula (I) from the environment. The shell is made of material which is able to release the at least one compound of formula (I). In one embodiment, the shell is made of material which is able to release the compound of formula (I) upon breakage of the shell and/or by diffusion through the shell. A person skilled in the art is well aware of processes to prepare said microcapsules. The core of the core-shell microcapsule, in addition to compound of formula (I), may further comprise at least one liquid carrier as defined above and/or at least one perfuming co-ingredient as defined above. The core-shell microcapsules or the core-shell microcapsule slurry comprising at least one compound of formula (I) may be dispersed into at least one liquid carrier as defined above. Said liquid carrier may further comprise at least one compound of formula (I) and/or at least one perfuming co-ingredient as defined above.

According to a particular embodiment, the shell of the microcapsule comprises a material selected from the group consisting of polyurea, polyurethane, polyamide, polyester, poly(meth)acrylate (i.e. polyacrylate and/or polymethacrylate), polysiloxane, polycarbonate, polysulfonamide, polymers of urea and formaldehyde, melamine and formaldehyde, melamine and urea, or melamine and glyoxal and mixtures thereof. The shell can also be hybrid, namely organic-inorganic such as a hybrid shell composed of at least two types of inorganic particles that are cross-linked, or yet a shell resulting from the hydrolysis and condensation reaction of a polyalkoxysilane macro-monomeric composition.

According to a particular embodiment, the core-shell microcapsule(s) can be also derived by using different or more than one encapsulation method.

In a preferred embodiment, the shell of the microcapsules may be, each independently, selected from the group of aminoplast, polyamide, polyester, polyurea and polyurethane shells and mixtures thereof.

In a particular embodiment, the shell of the microcapsules comprises an aminoplast copolymer, such as melamine-formaldehyde or urea-formaldehyde or cross-linked melamine formaldehyde or melamine glyoxal.

In a particular embodiment, the shell of the microcapsules is polyurea-based made from, for example but not limited to, isocyanate-based monomers and amine-containing crosslinkers such as guanidine carbonate and/or guanazole. Certain polyurea microcapsules comprise a polyurea wall which is the reaction product of the polymerization between at least one polyisocyanate comprising at least two isocyanate functional groups and at least one reactant selected from the group consisting of an amine (for example a water-soluble guanidine salt and guanidine); a colloidal stabilizer or emulsifier; and an encapsulated perfume. However, the use of an amine can be omitted.

In a particular embodiment, the colloidal stabilizer includes an aqueous solution of between 0.1% and 0.4% of polyvinyl alcohol, between 0.6% and 1% of a cationic copolymer of vinylpyrrolidone and of a quaternized vinylimidazole (all percentages being defined by weight relative to the total weight of the colloidal stabilizer). In a particular embodiment, the emulsifier is an anionic or amphiphilic biopolymer, which may be for example chosen from the group consisting of gum Arabic, soy protein, gelatin, sodium caseinate and mixtures thereof.

In a particular embodiment, the shell of the microcapsules is polyurethane-based made from, for example but not limited to, polyisocyanate and polyols, polyamide, polyester, etc.

In a particular embodiment, the microcapsules have a polymeric shell resulting from complex coacervation wherein the shell is possibly cross-linked.

In a particular embodiment of the core-shell microcapsules, the core-shell microcapsules comprise an oil-based core comprising a hydrophobic active, preferably at least one compound of formula (I), and a composite shell comprising a first material and a second material, wherein the first material and the second material are different, the first material is a coacervate, the second material is a polymeric material.

In a particular embodiment, the weight ratio between the first material and the second material is comprised between 50:50 and 99.9:0.1.

In a particular embodiment, the coacervate comprises a first polyelectrolyte, preferably selected among proteins (such as gelatin), polypeptides or polysaccharides (such as chitosan), most preferably gelatin, and a second polyelectrolyte, preferably alginate salts, cellulose derivatives, guar gum, pectinate salts, carrageenan, polyacrylic and methacrylic acid or xanthan gum, or yet plant gums such as acacia gum (Gum Arabic), most preferably Gum Arabic.

The first coacervate material can be hardened chemically using a suitable cross-linker such as glutaraldehyde, glyoxal, formaldehyde, tannic acid or genipin or can be hardened enzymatically using an enzyme such as transglutaminase.

The second polymeric material can be selected from the group consisting of polyurea, polyurethane, polyamide, polyester, polyacrylate, polysiloxane, polycarbonate, polysulfonamide, polymers of urea and formaldehyde, melamine and formaldehyde, melamine and urea, or melamine and glyoxal and mixtures thereof, preferably polyurea and/or polyurethane. The second material is preferably present in an amount less than 3 wt.%, preferably less than 1 wt.% based on the total weight of the microcapsule slurry.

The preparation of an aqueous dispersion/slurry of core-shell microcapsules is well known by a skilled person in the art. In a particular embodiment, the microcapsule wall material may comprise any suitable resin and especially including melamine, glyoxal, polyurea, polyurethane, polyamide, polyester, etc. Suitable resins include the reaction product of an aldehyde and an amine, suitable aldehydes include, formaldehyde and glyoxal. Suitable amines include melamine, urea, benzoguanamine, glycoluril, and mixtures thereof. Suitable melamines include, methylol melamine, methylated methylol melamine, imino melamine and mixtures thereof. Suitable urea include, dimethylol urea, methylated dimethylol urea, urea-resorcinol, and mixtures thereof. Suitable materials for making may be obtained from one or more of the following companies Solutia Inc. (St Louis, Missouri U.S.A.), Cytec Industries (West Paterson, New Jersey U.S.A.), Sigma-Aldrich (St. Louis, Missouri U.S.A.).

In a particular embodiment of the core-shell microcapsules, the core-shell microcapsules comprise
- an oil-based core comprising a hydrophobic active, preferably comprising at least one compound of formula (I),
- optionally an inner shell made of a polymerized polyfunctional monomer;
- a biopolymer shell comprising a protein, wherein at least one protein is cross-linked.

According to a particular embodiment, the protein is chosen from the group consisting of milk proteins, caseinate salts such as sodium caseinate or calcium caseinate, casein, whey protein, hydrolyzed proteins, gelatins, gluten, pea protein, soy protein, silk protein and mixtures thereof, preferably sodium caseinate, most preferably sodium caseinate.

According to a particular embodiment, the protein comprises sodium caseinate and a globular protein, preferably chosen from the group consisting of whey protein, betalactoglobulin, ovalbumine, bovine serum albumin, vegetable proteins, and mixtures thereof.

The protein is preferably a mixture of sodium caseinate and whey protein.

According to a particular embodiment, the biopolymer shell comprises a crosslinked protein chosen from the group consisting of sodium caseinate and/or whey protein.

According to a particular embodiment, the microcapsule slurry comprises at least one microcapsule made of:
- an oil-based core comprising the hydrophobic active, preferably comprising at least one compound of formula (I);
- an inner shell made of a polymerized polyfunctional monomer; preferably a polyisocyanate having at least two isocyanate functional groups;
- a biopolymer shell comprising a protein, wherein at least one protein is cross-linked; wherein the protein contains preferably a mixture comprising sodium caseinate and a globular protein, preferably whey protein;
- optionally at least an outer mineral layer.

According to an embodiment, sodium caseinate and/or whey protein is (are) crosslinked protein(s).

The weight ratio between sodium caseinate and whey protein is preferably comprised between 0.01 and 100, preferably between 0.1 and 10, more preferably between 0.2 and 5.

In a particular embodiment, the microcapsule is a one-shell aminoplast core-shell microcapsule obtainable by a process comprising the steps of:
1) admixing a perfume oil with at least a polyisocyanate having at least two isocyanate functional groups to form an oil phase;
2) dispersing or dissolving into water an aminoplast resin and optionally a stabilizer to form a water phase;
3) preparing an oil-in-water dispersion, wherein the mean droplet size is comprised between 1 and 100 microns, by admixing the oil phase and the water phase;
4) performing a curing step to form the wall of said microcapsule; and
5) optionally drying the final dispersion to obtain the dried core-shell microcapsule.

In a particular embodiment, the core-shell microcapsule is a formaldehyde-free capsule. A typical process for the preparation of an aminoplast formaldehyde-free microcapsule slurry comprises the steps of
1) preparing an oligomeric composition comprising the reaction product of, or obtainable by reacting together:
   a. a polyamine component in the form of melamine or of a mixture of melamine and at least one C₁-C₄ compound comprising two NH₂ functional groups;
   b. an aldehyde component in the form of a mixture of glyoxal, a C₄₋₆ 2,2-dialkoxy-ethanal and optionally a glyoxalate, said mixture having a molar ratio glyoxal/C₄₋₆ 2,2-dialkoxy-ethanal comprised between 1/1 and10/1; and
   c. a protic acid catalyst;
2) preparing an oil-in-water dispersion, wherein the droplet size is comprised between 1 and 600 microns, and comprising:
   a. an oil;
   b. a water medium;
   c. at least an oligomeric composition as obtained in step 1;
   d. at least a cross-linker selected amongst:
      i. C₄-C₁₂ aromatic or aliphatic di- or tri-isocyanates and their biurets, triurets, trimmers, trimethylol propane-adduct and mixtures thereof; and/or
      ii. a di- or tri-oxiran compound of formula:

         Q-(oxiran-2-ylmethyl)ₘ

         wherein m is 2 or 3 and Q represents a C₂-C₆ group optionally comprising from 2 to 6 nitrogen and/or oxygen atoms;
   e. optionally a C₁-C₄ compounds comprising two NH₂ functional groups;
3) heating the dispersion; and
4) cooling the dispersion.

The above process is described in more detail in WO 2013/068255.

In a particular embodiment of the core-shell microcapsules, the core-shell microcapsule is a polyamide core-shell microcapsule comprising:
- an oil based core comprising an hydrophobic active, preferably comprising at least one compound of formula (I), and
- a polyamide shell comprising or being obtainable from:
   - an acyl chloride,
   - a first amino compound, and
   - a second amino compound.

According to a particular embodiment, the polyamide core-shell microcapsule comprises:
an oil based core comprising an hydrophobic active, preferably comprising at least one compound of formula (I), and
a polyamide shell comprising or being obtainable from:
   - an acyl chloride, preferably in an amount comprised between 5 and 98%, preferably between 20 and 98%, more preferably between 30 and 85% w/w;
   - a first amino compound, preferably in an amount comprised between 1% and 50% w/w, preferably between 7 and 40% w/w;
   - a second amino compound, preferably in an amount comprised between 1% and 50% w/w, preferably between 2 and 25% w/w;
   - a stabilizer, preferably a biopolymer, preferably in an amount comprised between 0 and 90%, preferably between 0.1 and 75%, more preferably between 1 and 70%.

According to a particular embodiment, the polyamide core-shell microcapsule comprises:
- an oil based core comprising a hydrophobic active, preferably comprising at least one compound of formula (I), and
- a polyamide shell comprising or being obtainable from:
   - an acyl chloride,
   - a first amino-compound being an amino-acid, preferably chosen from the group consisting of L-Lysine, L-Arginine, L-Histidine, L-Tryptophane and/or a mixture thereof.
   - a second amino. compound chosen from the group consisting of ethylene diamine, diethylene triamine, cystamine and/or a mixture thereof, and
   - a biopolymer chosen from the group consisting of casein, sodium caseinate, bovin serum albumin, whey protein, and/or a mixture thereof.

The first amino-compound can be different from the second amino-compound. Typically, a process for preparing a polyamide-based microcapsule includes the following steps:
a) dissolving at least one acyl chloride in a hydrophobic material, preferably a perfume to form an oil phase;
b) dispersing the oil phase obtained in step a) into a water phase comprising a first amino compound to form an oil-in water emulsion;
c) performing a curing step to form polyamide microcapsules in the form of a slurry; wherein a stabilizer is added in the oil phase and/or in the water phase, and wherein at least a second amino-compound is added in the water phase before the formation of the oil-in-water emulsion and/or in the oil-in water emulsion obtained after step b).

In a particular embodiment, the shell of the microcapsule is polyurea-or polyurethane-based. Examples of processes for the preparation of polyurea and polyureathane-based microcapsule slurries are for instance described in WO 2007/004166, EP 2300146, and EP 2579976. Typically a process for the preparation of polyurea or polyurethane-based microcapsule slurries include the following steps:
a) dissolving at least one polyisocyanate having at least two isocyanate groups in an oil to form an oil phase;
b) preparing an aqueous solution of an emulsifier or colloidal stabilizer to form a water phase;
c) adding the oil phase to the water phase to form an oil-in-water dispersion, wherein the mean droplet size is comprised between 1 and 500 µm, preferably between 5 and 50 µm; and
d) applying conditions sufficient to induce interfacial polymerization and form microcapsules in form of a slurry.

In a particular embodiment, the microcapsule can be in form of a powder, which in particular may be obtained by submitting the microcapsule slurry to a drying step, like spray-drying, to provide the microcapsules as such, i.e. in a powdery form. It is understood that any standard method known by a person skilled in the art to perform such drying is also applicable. In particular the slurry may be spray-dried, preferably in the presence of a polymeric carrier material such as polyvinyl acetate, polyvinyl alcohol, dextrins, natural or modified starch, gum Arabic, vegetable gums, pectins, xanthans, alginates, carrageenans or cellulose derivatives to provide microcapsules in a powder form.

However, one may also cite other drying methods such as extrusion, plating, spray granulation or fluidized bed processes, or even drying at room temperature using materials (carrier, desiccant) that meet specific criteria as disclosed in WO 2017/134179.

According to a particular embodiment, the compositions mentioned above, comprise more than one compound of formula (I) and enable the perfumer to prepare accords or perfumes possessing the odor tonality of various compounds of the invention, creating thus new building block for creation purposes.

For the sake of clarity, it is also understood that any mixture resulting directly from a chemical synthesis, e.g. a reaction medium without an adequate purification, in which the compound of the invention would be involved as a starting, intermediate or end-product could not be considered as a perfuming composition according to the invention as far as said mixture does not provide the inventive compound in a suitable form for perfumery. Thus, unpurified reaction mixtures are generally excluded from the present invention unless otherwise specified.

The invention's compound can also be advantageously used in all the fields of modern perfumery, i.e. fine or functional perfumery, to positively impart or modify the odor of a consumer product into which said compound (I) is added. Consequently, another object of the present invention consists of a perfumed consumer product comprising, as a perfuming ingredient, at least one compound of formula (I), as defined above.

The invention's compound can be added as such or as part of an invention's perfuming composition.

For the sake of clarity, "perfumed consumer product" is meant to designate a consumer product which delivers at least a pleasant perfuming effect to the surface or space to which it is applied (e.g. skin, hair, textile, or home surface). In other words, a perfumed consumer product according to the invention is a perfumed consumer product which comprises a functional formulation, as well as optionally additional benefit agents, corresponding to the desired consumer product, and an olfactive effective amount of at least one invention's compound. For the sake of clarity, said perfumed consumer product is a non-edible product.

The nature and type of the constituents of the perfumed consumer product do not warrant a more detailed description here, which in any case would not be exhaustive, the skilled person being able to select them on the basis of his general knowledge and according to the nature and the desired effect of said product.

Non-limiting examples of suitable perfumed consumer products include a perfume, such as a fine perfume, a splash or eau de parfum, a cologne or a shave or after-shave lotion; a fabric care product, such as a liquid or solid detergent optionally in the form of a pod or tablet, a fabric softener, a liquid or solid scent booster, a dryer sheet, a fabric refresher, an ironing water, a paper, a bleach, a carpet cleaner, a curtain-care product; a body-care product, such as a hair care product (e.g. a shampoo, a leave-on or rinse-off hair conditioner, a coloring preparation or a hair spray, a color-care product, a hair shaping product, a dental care product), a disinfectant, an intimate care product; a cosmetic preparation (e.g. a skin cream or lotion, a vanishing cream or a deodorant or antiperspirant (e.g. a spray or roll on), a hair remover, a tanning or sun or after sun product, a nail product, a skin cleansing, a makeup); or a skin-care product (e.g. a soap, a shower or bath mousse, oil or gel, or a hygiene product or a foot/hand care products); an air care product, such as an air freshener or a "ready to use" powdered air freshener which can be used in the home space (rooms, refrigerators, cupboards, shoes or car) and/or in a public space (halls, hotels, malls, etc..); or a home care product, such as a mold remover, a furnisher care product, a wipe, a dish detergent or a hard-surface (e.g. a floor, bath, sanitary or a window-cleaning) detergent; a leather care product; a car care product, such as a polish, a wax or a plastic cleaner.

According to any embodiments of the invention, the perfumed consumer product of the invention are characterized by a pH of 1 or more. Particularly, the perfumed consumer product of the invention has a pH comprised between 1 and 12, or between 1 and 8. Even more particularly, the perfumed consumer product of the invention has a pH comprised between 1 and 6.

According to a particular embodiment, the invention's perfumed consumer product is in the form of a personal care, a home care or fabric care consumer product comprising ingredients that are common in the personal, home or fabric care consumer products, in particular shower gel, shampoo, soap, fabric detergents or softeners and all-purpose cleaners. The main functional constituents of perfumed consumer products are surfactants and/or softener components capable of cleaning and/or softening fabrics and/or textiles of varied nature, such as clothes, curtain fabrics, carpet and furniture fabrics, etc, or other home surfaces, skin or hair, and typically used in a large amount of water or water-based solvents. These are therefore formulations wherein the amount of water is typically comprised between 50 and 99% by weight of the perfumed consumer product with the exception of soap or solid detergent wherein the amount of water is at most 20%.

A more detailed description of such fabric cleaning and/or softening formulations is not warranted here, many descriptions of current liquid formulations can be found in the cleaner/fabric softener's patent and other pertinent literature, such as for example the textbook of Louis Ho Tan Tai, "Détergents et Produits de Soins Corporels, Chapters 1 to 7 in particular, Dunod, Paris, 1999, or any other similar and/or more recent textbooks pertaining to the art of liquid softener and all-purpose cleaners formulations. A patent publication, WO 2010/105873, is also cited by way of example, in as much as it describes typical current ingredients, other than perfumes, of such liquid products, particularly in pages 9 to 21. Of course, many other examples of liquid cleaner and/or fabric softener formulations can be found in the literature. Any such liquid formulations, namely liquid fabric cleaner or conditioner and/or all-purpose cleaner, can be used in the here-described compositions.

According to a particular embodiment of the invention, the invention's perfumed consumer product is a liquid fabric softener comprising a fabric softener active base in amount comprised between 85 and 100% by weight, based on the total weight of the perfumed consumer product. The main constituent of the fabric softener active base is water or water-based solvents. The fabric softener active base may comprise dialkyl quaternary ammonium salts, dialkyl ester quaternary ammonium salts, Hamburg esterquat, triethanolamine quat, silicones and mixtures thereof. Optionally, the fabric softener active base may further comprise a viscosity modifier in an amount comprised between 0.05 and 1% by weight, based on the total weight of the liquid base; preferably chosen in the group consisting of calcium chloride.

According to a particular embodiment of the invention, the invention's perfumed consumer product is an all-purpose cleaner comprising an all-purpose cleaner active base in amount comprised between 85 and 100% by weight, based on the total weight of the perfumed consumer product. The main constituent of the all-purpose cleaner active base is water or water-based solvents The all-purpose active base may comprise linear alkylbenzene sulfonates (LAS) in an amount comprised between 1 and 2%, nonionic surfactant in an amount comprised between 2 and 4% and acid such as citric acid in an amount comprised between 0.1 and 0.5%.

According to a particular embodiment of the invention, the invention's perfumed consumer product is a liquid detergent comprising a liquid detergent active base in amount comprised between 85 and 100% by weight, based on the total weight of the perfumed consumer product. The main constituent of the liquid detergent active base is water or water-based solvents. The liquid detergent active base may comprise anionic surfactant such as alkylbenzenesulfonate (ABS), linear alkylbenzene sulfonates (LAS), secondary alkyl sulfonate (SAS), primary alcohol sulfate (PAS), lauryl ether sulfate (LES), sodium lauryl ether sulfate (SLES), methyl ester sulfonate (MES); nonionic surfactant such as alkyl amines, alkanolamide, fatty alcohol poly(ethylene glycol) ether, fatty alcohol ethoxylate (FAE), ethylene oxide (EO) and propylene oxide (PO) copolymers, amine oxydes, alkyl polyglucosides, alkyl polyglucosamides; ormixtures thereof.

According to a particular embodiment of the invention, the invention's perfumed consumer product is a solid detergent comprising a solid detergent active base in amount comprised between 85 and 100% by weight, based on the total weight of the perfumed consumer product. The solid detergent active base may comprise at least one surfactant chosen in the group consisting of anionic, nonionic, cationic, zwiterionic surfactant and mixtures thereof. The surfactant in the solid detergent active base is preferably chosen in the group consisting of linear alkene benzene sulphonate (LABS), sodium laureth sulphate, sodium lauryl ether sulphate (SLES), sodium lauryl sulphate (SLS), alpha olefin sulphonate (AOS), methyl ester sulphonates (MES), alkyl polyglyucosides (APG), primary alcohol ethoxylates and in particular lauryl alcohol ethoxylates (LAE), primary alcohol sulphonates (PAS), soap and mixtures thereof. The soild detergent active base may comprise a further component, commonly used in powder detergent consumer product, selected from the group consisting of bleaching agents such as TAED (tetraacetylethylenediamine); buffering agent; builders such as zeolites, sodium carbonate or mixture thereof; soil release or soil suspension polymers; granulated enzyme particles such as cellulase, lipase, protease, mannanase, pectinase or mixtures thereof; corrosion inhibitor; antifoaming; sud suppressing agents; dyes; fillers such as sodium silicate, sodium sulfate or mixture thereof; source of hydrogen peroxide such as sodium percarbonate or sodium perborate; and mixtures thereof.

According to a particular embodiment of the invention, the invention's perfumed consumer product is shampoo or a shower gel comprising a shampoo or shower gel active base in amount comprised between 85 and 100% by weight, based on the total weight of the perfumed consumer product. The main constituent of the shampoo or a shower gel active base is water or water-based solvents The shampoo shower gel active base may comprise sodium alkylether sulfate, ammonium alkylether sulfates, alkylamphoacetate, cocamidopropyl betaine, cocamide MEA, alkylglucosides and aminoacid based surfactants.

According to a particular embodiment of the invention, the invention's perfumed consumer product is a soap bar comprising a soap active base in amount comprised between 85 and 100% by weight, based on the total weight of the perfumed consumer product. The soap bar active base may comprise salt of a weak acid, typically, a salt of weak acid, which may be a fatty acid and strong base like sodium hydroxide.

Some of the above-mentioned perfumed consumer products may represent an aggressive medium for the invention's compounds, so that it may be necessary to protect the latter from premature decomposition, for example by encapsulation or by chemically binding it to another chemical which is suitable to release the invention's ingredient upon a suitable external stimulus, such as an enzyme, light, heat or a change of pH.

The proportions in which the compounds according to the invention can be incorporated into the various aforementioned products or compositions vary within a wide range of values. These values are dependent on the nature of the article to be perfumed and on the desired organoleptic effect as well as on the nature of the co-ingredients in a given base when the compounds according to the invention are mixed with perfuming co-ingredients, solvents or additives commonly used in the art.

For example, in the case of perfuming compositions, typical concentrations are in the order of 0.001 % to 30 % by weight, or even more, of the compounds of the invention based on the weight of the composition into which they are incorporated. In the case of perfumed consumer product, typical concentrations are in the order of 0.0001 % to 10 % by weight, or even more, of the compounds of the invention based on the weight of the consumer product into which they are incorporated.

Unless specified otherwise, all percentages refer to percent by weight, based on the total weight of the referenced composition.

The invention's compounds can be prepared according to a method as described herein-below.

### Examples

The invention will now be described in further detail by way of the following examples, wherein the abbreviations have the usual meaning in the art, the temperatures are indicated in degrees centigrade (°C). NMR spectra were acquired using either a Bruker Avance II Ultrashield 400 plus operating at 400 MHz, (¹H) and 100 MHz (¹³C) or a Bruker Avance III 500 operating at 500 MHz (¹H) and 125 MHz (¹³C) or a Bruker Avance III 600 cryoprobe operating at 600 MHz (¹H) and 150 MHz (¹³C). Spectra were internally referenced relative to tetramethyl silane 0.0 ppm. ¹H NMR signal shifts are expressed in δ ppm, coupling constants (J) are expressed in Hz with the following multiplicities: s, singlet; d, doublet; t, triplet; q, quartet; m, multiplet; b, broad (indicating unresolved couplings) and were interpreted using Bruker Topspin software. ¹³C NMR data are expressed in chemical shift δ ppm and hybridization from DEPT 90 and DEPT 135 experiments, C, quaternary; CH, methine; CH₂, methylene; CH₃, methyl.

### Example 1

### Synthesis of a mixture comprising 1-(3,3-dimethylcyclopent-1-en-1-yl)ethan-1-ol and 1-(4,4-dimethylcyclopent-1-en-1-yl)ethan-1-ol

### a) Step 1: Preparation of 3,3-dimethylhexanedioic acid

3-neck 500 mL round bottomed flask equipped with a magnetic stir bar and reflux condenser was charged with 230 mg of VO₃NH₄ and 215g of 60% HNO₃. The solution was heated to 50 °C then carefully charged with 75g of 4,4,-dimethylcyclohexanol with a syringe pump: first, a few droplets of 4,4-dimethylcyclohexanol were added to the pot and the evolution of a red gas was observed nearly instantaneously. Once the evolution of red NOₓ gas was observed, the rest of the 4,4,-dimethylcyclohexanol was added over a 5h period during which the temperature rose as high as 60 °C. After the addition was complete, the temperature fell to 48 °C. At this point the reaction mixture was heated to 90-95 °C for 1h in order to blow off the remaining NOx. The green solution was then allowed to slowly cool to room temperature overnight. The following morning, white precipitate was observed and the slurry was further cooled to 0 °C with an ice bath. The solid was filtered, washed with cold water then dried at 0.06 mbar/75 °C to give 64.5g of 3,3-dimethylhexanedioic acid as a white powder (97% pure, silylated with MSTFA). The mother liquor was concentrated to 115g (from 440g) on the rotovap and allowed to slowly crystallize at room temperature, followed by cooling in an ice water bath to give an additional 9.3g of a 2nd crop of 3,3-dimethylhexanedioic acid at 96% purity. Yield = 70% The ¹H and ¹³C NMR spectra of the product were consistent with the literature spectra *(*Org. Lett. 2004, 6, 4411-4414)

### b) Step 2: Preparation of 3,3-dimethylcyclopentan-1-one

100 mL cylindrical kuegel-rohr flask was charged with 34.8g of 3,3-dimethylhexanedioic acid and 1.62g of Ba(OH)₂. To the cylindrical flask, two other kuegel-rohr bulbs were attached in series and kept outside the oven. The apparatus was rotated and the oven was heated to 270 °C at atmospheric pressure under a stream of N₂. Upon reaching 265 °C, water vapor, CO₂ as well as another volatile distillate began to be collected. After 2.5h, the oven temperature was raised to 275 °C and kept at this temperature for another 6.5h until no further distillate was observed. The oven was cooled and the distillate was phase separated and the aqueous phase was extracted twice with ether, combined, washed with NaHCO₃ and dried over Na₂SO₄. After removing the ether, 15.78g of clear yellow oil was obtained at 99% purity.

Yield of 3,3-dimethylcyclopentan-1-one = 90% based on 82% conversion of 3,3-dimethylhexanedioic acid.

The ¹H and ¹³C NMR spectra of the product were consistent with the literature spectra (J. Org. Chem. 1996, 61, 8885-8896)

### c) Step 3 : Preparation of 1-ethynyl-3, 3-dimethylcyclopentan-1-ol

250mL 3-neck round bottom flask equipped with magnetic stir bar and reflux condenser was charged under N₂ with 130 mL of a 0.5M solution of ethynyl-MgBr in THF and cooled to 2 °C. 5g of 3,3-dimethylcyclopentan-1-one was then added via syringe pump over a 5 min period causing the temperature to rise to 8 °C. The reaction was allowed to reach room temperature and stirred for a total of 16.5h. The reaction was quenched at 20 °C with 26g of 10% HCl. The phases were separated and the aq. phase was extracted 3x with ether, combined, washed with NaHCO₃, dried over Na₂SO₄ and concentrated on the rotovap then distilled on the kuegel-rohr (15-10 mbar/100-150 °C) to give 5.1g of 97% 1-ethynyl-3,3-dimethylcyclopentan-1-ol (84% yield)
¹H-NMR (600MHz, CDCl₃): δ (ppm) 1.07 (s, 3H), 1.13 (s, 3H), 1.55-2.18 (m, 7H), 2.49 (s, 1H)
¹³C-NMR (150MHz, CDCl₃): δ (ppm) 88.53, 77.23, 77.02, 76.81, 74.93, 70.92, 56.95, 42.46, 39.52, 38.56, 30.76, 30.39

### d) Step 4 : Preparation of a mixture comprising 1-(3,3-dimethylcyclopent-1-en-1-yl)ethan-1-one and 1-(4, 4-dimethylcyclopent-1-en-1- yl)ethan-1-one

2000 mL 3-neck flask equipped a magnetic stir bar and reflux condenser was placed under N₂ atmosphere and charged with 375g of 96% H₂SO₄, 700 mL of heptane and cooled to - 25 °C. Using a syringe pump, 87g of 1-ethynyl-3,3-dimethylcyclopentan-1-ol was introduced over a 2.5h period. During the addition the temperature varied from -25 to -17 °C. After the introduction of 1-ethynyl-3,3-dimethylcyclopentan-1-ol, GC analysis of the reaction mixture showed complete conversion. The dark mixture was poured into ice. The aqueous phase was extracted with ether, washed with NaHCO₃, dried over Na₂SO₄ and concentrated on the rotovap to give 79.1g of crude concentrate. The latter was then distilled on a short Vigreux column to give 70.3g of a mixture comprising 1-(3,3-dimethylcyclopent-1-en-1-yl)ethan-1-one (76%) and 1-(4,4-dimethylcyclopent-1-en-1-yl)ethan-1-one (20%), 80% yield for the mixture of enones 1-(3,3-dimethylcyclopent-1-en-1-yl)ethan-1-one and 1-(4,4-dimethylcyclopent-1-en-1-yl)ethan-1-one.
¹H-NMR (600MHz, CDCl₃): δ (ppm) 1.10 (s, 6H, minor regioisomer), 1.13 (s, 6H, major regioisomer), 1.70 (s, 2H, minor regioisomer), 1.74 (t, 2H, *J* 6 Hz, major regioisomer), 2.299 (s, 3H, major regioisomer), 2.302 (s, 3H, minor regioisomer), 2.38 (m, 2H, minor regioisomer), 2.56 (triplet of doublets, 2H, *J₁* 6 Hz, *J₂* 1.8 Hz, major regioisomer), 6.45 (t, 1H, *J* 1.8 Hz, minor regioisomer), 6.62 (bs, 1H, major regioisomer)
¹³C-NMR (150MHz, CDCl₃): δ (ppm), Major regioisomer only,142.8, 153.6, 197.6, 77.2, 77.0, 76.8, 46.4, 38.4, 29.6, 29.5, 27.5

### e) Step 5 : Preparation of a mixture comprising 1-(3,3-dimethylcyclopent-1-en-1-yl)ethan-1-ol and 1-(4,4-dimethylcyclopent-1-en-1-yl)ethan-1-ol

3-neck 1000 mL round bottomed flask equipped with a magnetic stir bar and reflux condenser was placed under N₂ atmosphere and charged with 9.4g of LiAlH₄, followed by 500 mL of anhydrous ether. The slurry was cooled to 4 °C then introduced with 70g of mixture comprising 1-(3,3-dimethylcyclopent-1-en-1-yl)ethan-1-one and 1-(4,4-dimethylcyclopent-1-en-1-yl)ethan-1-one as prepared in Step 4 over a 2h period causing the temperature to rise to a maximum of 7 °C. At the end of the introduction, no starting material remained. The reaction was quenched at 0 °C with the careful addition of 9.4 mL of H₂O, followed by 9.4 mL of 5% NaOH and 28 mL of water. The slurry was stirred until it became white-grey in color than filtered on a frit containing celite. After concentrating the filtrate, 67.8g of a crude mixture of 1-(3,3-dimethylcyclopent-1-en-1-yl)ethan-1-ol and 1-(4,4-dimethylcyclopent-1-en-1-yl)ethan-1-ol was obtained and used as is in the next step without further purification.

### Example 2

### Synthesis of a mixture comprising 2-(1-(3,3-dimethylcyclopent-1-en-1-yl)ethoxy)-2-methylpropan-1-ol and 2-(1-(4,4-dimethylcyclopent-1-en-1-yl)ethoxy)-2-methylpropan-1-ol

25 mL 3-neck round bottomed flask equipped with a magnetic stir bar and reflux condenser was charged with 10g of a mixture comprising 1-(3,3-dimethylcyclopent-1-en-1-yl)ethan-1-ol and 1-(4,4-dimethylcyclopent-1-en-1-yl)ethan-1-ol as prepared in Example 1, 4 mL of cyclohexane and cooled to 0 °C under N₂. 0.12g of BF₃OEt₂ was then added in one shot causing the temperature to rise to 1.5 °C. 1.23g of i-butyleneoxide dissolved in 0.83g of cyclohexane was then added with a over a 3h period, then stirred for an additional 20 min before quenching with NaHCO₃. The aqueous phase was extracted 3x with ether, combined, dried over Na₂SO₄ and concentrated on the rotovap to give 11.73g of crude. The crude was distilled on a Fischer column to give 7.62g of unreacted 1-(3,3-dimethylcyclopent-1-en-1-yl)ethan-1-ol and 1-(4,4-dimethylcyclopent-1-en-1-yl)ethan-1-ol and 1.49g of a mixture comprising 2-(1-(3,3-dimethylcyclopent-1-en-1-yl)ethoxy)-2-methylpropan-1-ol and 2-(1-(4,4-dimethylcyclopent-1-en-1-yl)ethoxy)-2-methylpropan-1-ol (50% yield).
¹H-NMR (600MHz, CDCl₃): δ (ppm) Major regioisomer only 1.02 (s, 3H), 1.03 (s, 3H), 1.14 (s, 3H), 1.18 (s, 3H), 1.20 (d, 3H, *J* 6 Hz), 1.68 (t, 1H, *J* 8 Hz), 2.33 (t, 1H, *J* 8 Hz), 3.34 (d, 1H, *J* 13 Hz), 3.40 (d, 1H, *J* 13 Hz), 4.20 (q, 1H, *J* 8 Hz), 5.33 (s, 1H)
¹³C-NMR (150MHz, CDCl₃): δ (ppm), Major regioisomer only, 145.6, 135.0, 77.2, 77.0, 76.8, 75.9, 69.9, 66.7, 44.5, 39.2, 30.5, 28.3, 28.2, 23.0, 22.5, 22.4

### Example 3

### Synthesis of compounds of formula (I) and comparative examples

### a) Preparation of a composition of matter comprising 2-[1-(3,3-dimethyl-1-cyclopenten-1-yl)ethoxy]-2-methylpropyl propionate and 2-[1-(4,4-dimethyl-1-cyclopenten-1-yl)ethoxy]-2-methylpropyl propionate (compounds of formula (I))

3-neck 100 mL round bottomed flask equipped with a magnetic stir bar and reflux condenser was charged with 15.4g of a mixture comprising 2-(1-(3,3-dimethylcyclopent-1-en-1-yl)ethoxy)-2-methylpropan-1-ol and 2-(1-(4,4-dimethylcyclopent-1-en-1-yl)ethoxy)-2-methylpropan-1-ol as prepared in example 2, 0.42g of DMAP and 50 mL of toluene. The solution was heated under N₂ to 50 °C then introduced with 12.2g of propionic anhydride in one shot causing the reaction temperature to rise to 66 °C within 5 min. The reaction was stirred at this temperature for 1h to complete conversion of the starting material. The reaction mixture was then slowly poured into 128g of saturated NaHCO₃ and stirred vigorously for 2h before transferring it to a separatory funnel. The phases were separated (pH 8) and the aqueous phase was extracted 2x with toluene, combined and concentrated to give 20.51g of crude concentrate. The latter was distilled on the kuegel-rohr to give 20g of a composition of matter comprising 2-[1-(3,3-dimethyl-1-cyclopenten-1-yl)ethoxy]-2-methylpropyl propionate and 2-[1-(4,4-dimethyl-1-cyclopenten-1-yl)ethoxy]-2-methylpropyl propionate in a ratio of 83/17. Yield = 97%.
For NMR data see Example 3b) Step 5

### b) Preparation of a composition of matter comprising exclusively 2-[1-(3,3-dimethyl-1-cyclopenten-1-yl)ethoxy]-2-methylpropyl propionate (compounds of formula (I))

### Step 1- Preparation of 2,2-dimethylhexanedial

1.9g of 2,2-dimethylpent-4-enal prepared from the procedure described in Org. Synth. Coll. Vol. 7, 1990, 177 was hydroformylated at 90 °C in an autoclave using 0.015g of Hydridocarbonyltris(triphenylphosphine)rhodium (I), 20 mL of ethyl acetate and 20 bars of CO/H₂ to give a mixture containing 48% 2,2-dimethylhexanedial (linear product) and 35% 2,2,4-trimethylpentanedial (branched product). After solvent removal, the crude was chromatographed on silica using a pentane/ether gradient of 99/1, 95/5, 90/10, 80/20 to 70/30 to give 1.3g of 85% pure 2,2-dimethylhexanedial after a bulb-to-bulb distillation of the chromatographed material. The ¹H and ¹³C NMR spectra of the product were consistent with the literature spectra *(*J.Am.Chem.Soc. 2005, 127, 16778 - 16779).

### Step 2- Preparation of 3,3-dimethylcyclopent-1-ene-1-carbaldehyde

Following the procedure described in Org. Synth. Coll. Vol. 8, 1993, 208, 0.89g of crude 3,3-dimethylcyclopent-1-ene-1-carbaldehyde (74% pure) was prepared using 1.1g of 2,2-dimethylhexanedial and used without further purification in the subsequent step.

### Step 3- Preparation of 1-(3,3-dimethylcyclopent-1-en-1-yl)ethan-1-ol

A 25 mL 3-neck round bottomed flask equipped with a magnetic stir bar and reflux condenser was charged with 2.3mL of 3*M* MeMgI and cooled to 0 °C under N₂ atmosphere. To the Grignard, 890mg of 3,3-dimethylcyclopent-1-ene-1-carbaldehyde (74%) prepared in step 2, dissolved in about 10 mL of ether was added in one shot causing the pot temperature to rise from 4-15 °C. The reaction was allowed to reach 20 °C and stirred for a total of 1.5h then quenched with 10% HCl. The crude was diluted with ether, transferred to a 50 mL separatory funnel, phase separated, extracted 2x with ether, washed with NaHCO₃, dried over Na₂SO₄ and concentrated on the rotovap to give 1.1g of crude yellow oil. The latter was chromatographed on silica using 8/2 pentane/ether to give 1g of 99% pure 1-(3,3-dimethylcyclopent-1-en-1-yl)ethan-1-ol. Following bulb-to-bulb distillation, 670mg of 99% 1-(3,3-dimethylcyclopent-1-en-1-yl)ethan-1-ol was obtained.
¹H-NMR (600MHz, CDCl₃): δ (ppm) 1.038 (s, 3H), 1.044 (s, 3H), 1.29 (d, 3H, *J* 6.6 Hz), 1.70 (t, 2H, *J* 7.2 Hz), 2.35 (t, 2H, *J* 7.2 Hz), 4.35 (bq, 1H), 5.35 (s, 1H)
¹³C-NMR (150MHz, CDCl₃): δ (ppm), 145.4, 134.8, 77.2, 77.0, 76.8, 67.2, 44.6, 39.2, 30.5, 28.37, 28.36, 22.1

### Step 4- Preparation of 2-(1-(3,3-dimethylcyclopent-1-en-1-yl)ethoxy)-2-methylpropan-1-ol

Using pure 1-(3,3-dimethylcyclopent-1-en-1-yl)ethan-1-ol, and the procedure described in Example 2 above, 2-(1-(3,3-dimethylcyclopent-1-en-1-yl)ethoxy)-2-methylpropan-1-ol was prepared.

### Step 5- Preparation of 2-[1-(3,3-dimethyl-1-cyclopenten-1-yl)ethoxy]-2-methylpropyl propionate

Using pure 2-(1-(3,3-dimethylcyclopent-1-en-1-yl)ethoxy)-2-methylpropan-1-ol, and the procedure described in Example 3a above, 2-[1-(3,3-dimethyl-1-cyclopenten-1-yl)ethoxy]-2-methylpropyl propionate was prepared.
¹H-NMR (600MHz, CDCl₃): δ (ppm) 1.010 (s, 3H), 1.018 (s, 3H), 1.14-1.19 (m, 12H), 1.66 (t, 2H, *J* 8.6 Hz), 2.32 (t, 2H, *J* 8.6 Hz), 2.37 (q, 2H, J9.1 Hz), 3.93 (d, 1H, *J* 13.5 Hz), 4.01 (d, 1H, *J* 13.5 Hz), 4.22 (q, 1H, *J* 7.7 Hz), 5.31 (s, 1H)
¹³C-NMR (150MHz, CDCl₃): δ (ppm), 174.3, 145.3, 134.8. 77.2, 77.0, 76.8, 74.4, 69.6, 67.1, 44.5, 39.2, 30.5, 28.3, 28.1, 27.7, 23.7, 23.6, 22.3, 22.1, 9.2

### c) Preparation of 2-(1-(3,3-dimethylcyclopentyl)ethoxy)-2-methylpropyl propionate

### (Comparative compound)

2.5g of 2-[1-(3,3-dimethyl-1-cyclopenten-1-yl)ethoxy]-2-methylpropyl propionate prepared in Example 3b was dissolved in 18 mL of THF and charged with 50mg of 5% Pd/C. The mixture was hydrogenated with 1 bar of H₂ overnight to complete conversion of the starting material into a 1/1 diastereomeric mixture of 2-(1-(3,3-dimethylcyclopentyl)ethoxy)-2-methylpropyl propionates. After filtering off the catalyst and removal of THF on the rotovap, 2.51g of concentrate was obtained. The latter was distilled on the kuegel-rohr to afford 2.5g of 2-(1-(3,3-dimethylcyclopentyl)ethoxy)-2-methylpropyl propionates (mixture of diastereomers, 99% pure)
¹H-NMR (600MHz, CDCl₃): δ (ppm) mixture of diastereomers 0.95 (s, 3H), 0.96 (s, 3H), 1.00 (d, 3H, *J* 2.6 Hz), 1.08 (d, 1H, *J* 6 Hz), 1.10 (d, 1H, *J* 6 Hz), 1.19 (s, 6H, *J* 7.5 Hz), 1.65-1.72 (m, 1H), 1.78-1.84 (m, 1H), 1.97-2.06 (m, 1H), 2.36 (q, 2H, *J* 7.6 Hz), 3.45 (bdt, 1H), 3.94 (bd, 2H)
¹³C-NMR (150MHz, CDCl₃): δ (ppm), mixture of diastereomers, 174.38, 174.37, 77.23, 77.02, 76.81, 73.72, 73.70, 72.2, 72.0, 70.53, 70.49, 46.9, 46.8, 45.001, 44.498, 40.56, 40.45, 38.7, 38.5, 30.3, 30.2, 29.5, 29.25, 29.00, 28.4, 27.7, 24.34, 24.32, 24.30, 23.97, 23.96, 22.0, 21.98, 9.2.

### d) Preparation of a composition of matter comprising 2-[1-(3,3-dimethyl-1-cyclopenten-1-yl)ethoxy]-2-oxoethyl propionate and 2-[1-(4,4-dimethyl-1-cyclopenten-1-yl)ethoxy]-2-oxoethyl propionate

0.5g of a mixture comprising 1-(3,3-dimethylcyclopent-1-en-1-yl)ethan-1-ol and 1-(4,4-dimethylcyclopent-1-en-1-yl)ethan-1-ol as prepared in Example 1, 0.49g methylchloro acetate, 0.072g octyl₂SnO and 10 mL cyclohexane were charged into 25 mL schlenk tube equipped with a mag stir bar and Dean-Stark trap and refluxed for 2h. After concentrating the reaction mixture on the rotovap, 0.9g of crude chloroesters 1-(3,3-dimethylcyclopent-1-en-1-yl)ethyl 2-chloroacetate and 1-(4,4-dimethylcyclopent-1-en-1-yl)ethyl 2-chloroacetate were obtained. The latter was re-dissolved into 10 mL of NMP and charged with 0.5g of sodium propionate, 0.05g of NaI and the slurry was stirred at 60 °C for 2h to complete conversion. Water was added to dissolve the solids and the orange mixture was extracted first with cyclohexane (3x) then ether (3x). The combined extracts were then washed with NaHCO₃, dried over Na₂SO₄ and concentrated on the rotovap. The crude concentrate was chromatographed on silica (60g, 10/1 heptane/EtOAc) to give 0.79g of 95% pure composition of matter comprising 2-[1-(3,3-dimethyl-1-cyclopenten-1-yl)ethoxy]-2-oxoethyl propionate and 2-[1-(4,4-dimethyl-1-cyclopenten-1-yl)ethoxy]-2-oxoethyl propionate at a ratio of 75/25, respectively. The latter was distilled on the kuegel-rohr to give 0.658g of clear white distillate at 98% purity.

¹H-NMR (600MHz, CDCl₃): δ (ppm) mixture of regioisomers 1.033 (s, 3H), 1.037 (s, 3H), 1.070 (d, 1H), 1.19 (t, 3H, *J* 7.6 Hz), 1.35 (d, 3H, *J* 10 Hz), 1.69 (t, 1H, *J* 7.2 Hz), 2.26-2.36 (m, 1H), 2.45 (q, 2H, *J* 7.6 Hz), 4.61 (s, 2H), 5.41 (s, 1H, major regioisomer), 5.51 (broad dd, 2H, minor regioisomer).

¹³C-NMR (150MHz, CDCl₃): δ (ppm), mixture of regioisomers, 173.78, 173.76, 167.33, 141.59, 140.13, 137.48, 125.72, 77.23, 77.02, 76.81, 70.87, 70.69, 60.76, 60.72, 47.29, 46.58, 44.79, 38.95, 38.52, 30.75, 29.67, 29.64, 28.19, 27.17, 18.99, 18.86, 8.99.

### e) Preparation of a composition of 2-(1-(3,3-dimethylcyclopentyl)ethoxy)-2-oxoethyl propionate (comparative compound)

### Step 1

3.4g of a mixture containing 1-(3,3-dimethylcyclopent-1-en-1-yl)ethan-1-ol and 1-(4,4-dimethylcyclopent-1-en-1-yl)ethan-1-ol was dissolved into 30 mL of THF and hydrogenated using 35mg of PtO₂ as the catalyst at room temperature with 1 bar of H₂. After filtering off the catalyst through a bed of celite and solvent removal, 3.5g of 95% pure 1-(3,3-dimethylcyclopentyl)ethan-1-ol was obtained and used as is in the next step without further purification.

### Step 2

A 25 mL 3-necked round bottomed flask equipped with a magnetic stir bar and a Dean-Stark trap was charged with 1.4g of crude 1-(3,3-dimethylcyclopentyl)ethan-1-ol prepared in step 1, 1.3g of chloroacetic acid, 0.038g of pTSA and 10 mL of toluene. The mixture was heated to reflux for 2h to complete conversion of the starting material into 2 diastereomeric esters of 1-(3,3-dimethylcyclopentyl)ethyl 2-chloroacetate at a ratio = 48/45. The dark brown solution was cooled to 25 °C and poured into 12g of saturated NaHCO₃ thereby turning the organic phase light orange in color. After stirring the mixture for 5 min, the contents were transferred to a separatory funnel and phase separated. The aqueous phase was extracted once with toluene, combined with the rest of the organic content and concentrated on the rotovap to give 1.9g of crude 1/1 diastereomeric mixture of 1-(3,3-dimethylcyclopentyl)ethyl 2-chloroacetates, which was used as is in the next step.

### Step 3

50 mL 3-necked round bottomed flask equipped with a magnetic stir bar and reflux condenser was charged with 1.8g of crude 1-(3,3-dimethylcyclopentyl)ethyl 2-chloroacetate prepared in step 2, 15g of NMP, 1.15g of sodium propionate and 0.12g of NaI. The slurry was stirred at 20 °C for 64h, then at 75 °C for 2h to complete conversion of the starting material into 88% of a 1/1 diastereomeric mixture of 2-(1-(3,3-dimethylcyclopentyl)ethoxy)-2-oxoethyl propionates. The reaction mixture was cooled to 25 °C, charged with 15 mL of water and transferred to a separatory funnel. The dark red solution was extracted 5x with MTBE and the combined extracts (light yellow) was washed 2x with 15% NaHSO₃ to completely remove color, followed by a NaHCO₃, dried over Na₂SO₄ and concentrated on the rotovap to give 1.88g of crude 2-(1-(3,3-dimethylcyclopentyl)ethoxy)-2-oxoethyl propionate. The latter was distilled on the kuegel-rohr to afford 0.96g, of 96% pure 2-(1-(3,3-dimethylcyclopentyl)ethoxy)-2-oxoethyl propionate as a 1/1 mixture of 2 diastereomers.
¹H-NMR (600MHz, CDCl₃): δ (ppm) mixture of diastereomers 0.96 (s, 3H), 0.97 (s, 3H), 1.01 (s, 3H), 1.02 (s, 3H), 1-17-1.22 (m, 6H), 1.31-1.55 (m, 4H), 1.73-1.82 (m, 1H), 2.16-2.23 (m, 1H), 2.45 (q, 2H, J7.3 Hz), 4.58 (s, 2H), 4.82-4.89 (m, 1H)
¹³C-NMR (150MHz, CDCl₃): δ (ppm), mixture of diastereomers, 173.79, 173.78, 167.72, 167.67, 77.24, 77.03, 76.81, 70.82, 76.34, 76.33, 60.81, 60.80, 44.35, 44.32, 43.94, 43.75, 40.43, 40.27, 39.07, 38.90, 29.83, 29.82, 28.92, 28.81, 27.92, 27.81, 27.18, 18.94, 18.86, 8.98.

### f) Preparation of a composition of matter comprising 2-[1-(3,3-dimethyl-1-cyclopenten-1-yl)ethoxy]-2-methylpropyl 2-butenoate and 2-[1-(4,4-dimethyl-1-cyclopenten-1-yl)ethoxy]-2-methylpropyl 2-butenoate

25 mL round bottomed flask equipped with a Dean-Stark trap was charged with 0.7g of 2-(1-(3,3-dimethylcyclopent-1-en-1-yl)ethoxy)-2-methylpropan-1-ol and 2-(1-(4,4-dimethylcyclopent-1-en-1-yl)ethoxy)-2-methylpropan-1-ol as prepared in example 2, 0.7g of ethyl crotonate, 0.04g of dibutyltin oxide, 10 mL of cyclohexane and heated to reflux under N₂ for 18h then cooled to 20 °C. The reaction mixture was concentrated on the rotovap to give 0.89g of crude. The latter was chromatographed on silica using 3/1 pentane/ether as the solvent to give 0.546g of 99% of a composition of matter comprising 2-[1-(3,3-dimethyl-1-cyclopenten-1-yl)ethoxy]-2-methylpropyl 2-butenoate and 2-[1-(4,4-dimethyl-1-cyclopenten-1-yl)ethoxy]-2-methylpropyl 2-butenoate. After distilling on the kuegel-rohr 0.529g of 99% of a composition of matter comprising 2-[1-(3,3-dimethyl-1-cyclopenten-1-yl)ethoxy]-2-methylpropyl 2-butenoate and 2-[1-(4,4-dimethyl-1-cyclopenten-1-yl)ethoxy]-2-methylpropyl 2-butenoate was obtained. Yield = 61%.
¹H-NMR (600MHz, CDCl₃): δ (ppm) mixture of regioisomers 1.00 (s, 3H), 1.01 (s, 3H), 1.19 (d, 1H, *J 6.5* Hz), 1.205 (s, 3H), 1.213, (s, 3H), 1.66 (t, 2H, *J* 7.1 Hz), 1.89 (d, 3H, *J* 6.8 Hz), 2.32 (t, 2H, *J* 7.1 Hz), 3.98 (d, 1H, *J* 11 Hz), 4.05 (d, 1H, *J* 11 Hz), 4.24 (q, 1H, *J* 6.4 Hz), 5.30 (s, 1H), 5.88 (d, 1H, *J* 15.6 Hz), 6.70 (dq, 1H, *J₁* 7 Hz, *J₂* 15.6 Hz)
¹³C-NMR (150MHz, CDCl₃): δ (ppm), mixture of regioisomers, 166.39, 145.28, 144.73, 134.81, 122.70, 77.22, 77.02, 76.81, 74.50, 69.65, 67.17, 44.51, 39.20, 30.52, 28.35, 28.18, 23.74, 23.72, 22.34, 17.99

### g) Preparation of a composition of matter comprising 2-[1-(3,3-dimethyl-1-cyclopenten-1-yl)ethoxy]-2-methylpropyl methoxyacetate and 2-[1-(4,4-dimethyl-1-cyclopenten-1-yl)ethoxy]-2-methylpropyl methoxyacetate

25 mL Schlenk tube equipped with a Dean-Stark trap was charged with 0.7g of 2-(1-(3,3-dimethylcyclopent-1-en-1-yl)ethoxy)-2-methylpropan-1-ol and 2-(1-(4,4-dimethylcyclopent-1-en-1-yl)ethoxy)-2-methylpropan-1-ol as prepared in example 2, 1g of methyl methoxyacetate, 0.04g of dibutyltin oxide, 10 mL of cyclohexane and heated to reflux under N₂ for 3h. The reaction mixture was cooled and concentrated on the rotovap to give 1.09g of concentrate. The concentrate was chromatographed on silica using 3/1 pentane/ether as the solvent to give 0.74g of a composition of matter comprising 2-[1-(3,3-dimethyl-1-cyclopenten-1-yl)ethoxy]-2-methylpropyl methoxyacetate and 2-[1-(4,4-dimethyl-1-cyclopenten-1-yl)ethoxy]-2-methylpropyl methoxyacetate which was distilled on the kuegel-rohr to give 0.73g of 99% of a composition of matter comprising 2-[1-(3,3-dimethyl-1-cyclopenten-1-yl)ethoxy]-2-methylpropyl methoxyacetate and 2-[1-(4,4-dimethyl-1-cyclopenten-1-yl)ethoxy]-2-methylpropyl methoxyacetate. Yield = 91%
¹H-NMR (600MHz, CDCl₃): δ (ppm) mixture of regioisomers 1.01 (s, 3H), 1.02 (s, 3H), 1.19 (d, 1H, *J* 6.5 Hz), 1.20 (s, 6H), 1.66 (t, 2H, *J* 7.1 Hz), 2.32 (t, 2H, *J* 6.9 Hz), 3.46 (s, 3H), 4.01 (d, 1H, *J* 11.2 Hz), 4.07 (s, 2H), 4.10 (d, 1H, *J* 11.2 Hz ), 4.22 (q, 1H, *J* 6.4 Hz), 5.30 (s, 1H)
¹³C-NMR (150MHz, CDCl₃): δ (ppm), mixture of regioisomers, 170.23, 145.23, 134.91, 77.23, 77.02, 76.81, 74.32, 70.28, 69.73, 67.22, 59.41, 44.53, 39.21, 30.46, 28.33, 28.17, 23.57, 23.49, 22.33

### h) Preparation of a composition of matter comprising 2-[1-(3,3-dimethyl-1-cyclopenten-1-yl)ethoxy]-2-methylpropyl ethyl carbonate and 2-[1-(4,4-dimethyl-1-cyclopenten-1-yl)ethoxy]-2-methylpropyl ethyl carbonate

25 mL 3-neck round bottomed flask equipped with a reflux condenser was charged with 0.6g of 2-(1-(3,3-dimethylcyclopent-1-en-1-yl)ethoxy)-2-methylpropan-1-ol and 2-(1-(4,4-dimethylcyclopent-1-en-1-yl)ethoxy)-2-methylpropan-1-ol as prepared in example 2, 0.016g of DMAP, 0.4g of triethylamine, 10 mL of toluene and cooled under N₂ to 15 °C in a water bath. The reaction mixture was then charged with 0.325 mL of Ethyl chloroformate in one shot causing the reaction temp to rise to 20 °C. After 1h45m, another 0.8 mL of ethyl chloroformate was added, followed by 0.6g of triethylamine. The slurry was stirred for a total of 69h then quenched with the addition of 10% HCl, transferred to a 50 mL separatory funnel and extracted with ether, washed with NaHCO₃, dried over Na₂SO₄ and rotovapped to give 0.69g of crude. The latter was chromatographed on silica using 5/1 pentane/ether as solvent to give 0.318g of 82% of a composition of matter comprising 2-[1-(3,3-dimethyl-1-cyclopenten-1-yl)ethoxy]-2-methylpropyl ethyl carbonate and 2-[1-(4,4-dimethyl-1-cyclopenten-1-yl)ethoxy]-2-methylpropyl ethyl carbonate. The chromatographed material was distilled on the kuegel-rohr to give 0.24g of 98% of a composition of matter comprising 2-[1-(3,3-dimethyl-1-cyclopenten-1-yl)ethoxy]-2-methylpropyl ethyl carbonate and 2-[1-(4,4-dimethyl-1-cyclopenten-1-yl)ethoxy]-2-methylpropyl ethyl carbonate. Yield = 32%
¹H-NMR (600MHz, CDCl₃): δ (ppm) mixture of regioisomers 1.01 (s, 3H), 1.02 (s, 3H), 1.19 (d, 1H, *J* 6.4 Hz), 1.22 (s, 6H), 1.31 (t, 3H, *J* 7 Hz), 1.66 (t, 2H, *J* 7.1 Hz), 2.32 (t, 2H, *J* 7 Hz), 3.96 (d, 1H, *J* 10.8 Hz), 4.03 (d, 1H, *J* 10.8 Hz), 4.20 (q, 2H, *J* 7.3 Hz), 4.25 (q, 1H, *J* 6 Hz), 5.30 (s, 1H)
¹³C-NMR (150MHz, CDCl₃): δ (ppm), mixture of regioisomers, 155.28, 145.30, 134.84, 77.23, 77.02, 76.80, 74.29, 73.48, 67.25, 63.95, 44.51, 39.20, 30.41, 28.31, 28.16, 23.56, 23.48, 22.35, 14.31

### i) Preparation of a composition of matter comprising 2-[1-(3,3-dimethyl-1-cyclopenten-1-yl)ethoxy]-2-methylpropyl (2R)-2-hydroxypropanoate and 2-[1-(4,4-dimethyl-1-cyclopenten-1-yl)ethoxy]-2-methylpropyl (2R)-2-hydroxypropanoate

25 mL 3-neck round bottomed flask equipped with a mini Dean-Stark trap was charged with 0.6g of 2-(1-(3,3-dimethylcyclopent-1-en-1-yl)ethoxy)-2-methylpropan-1-ol and 2-(1-(4,4-dimethylcyclopent-1-en-1-yl)ethoxy)-2-methylpropan-1-ol as prepared in example 2, 0.32g of L-ethyl lactate, 0.033g of dibutyltin oxide, 10 mL of cyclohexane and heated to reflux under N₂. After 40h, the reaction was cooled to room temperature. The reaction mixture was cooled and concentrated on the rotovap to give 1.11g of crude. The latter was chromatographed on silica using 2/1 pentane/ether as the solvent to give 0.4g of 92% of a composition of matter comprising 2-[1-(3,3-dimethyl-1-cyclopenten-1-yl)ethoxy]-2-methylpropyl (2R)-2-hydroxypropanoate and 2-[1-(4,4-dimethyl-1-cyclopenten-1-yl)ethoxy]-2-methylpropyl (2R)-2-hydroxypropanoate. The latter was distilled on the kuegel-rohr to give 0.365g of distillate containing 98% of a composition of matter comprising 2-[1-(3,3-dimethyl-1-cyclopenten-1-yl)ethoxy]-2-methylpropyl (2R)-2-hydroxypropanoate and 2-[1-(4,4-dimethyl-1-cyclopenten-1-yl)ethoxy]-2-methylpropyl (2R)-2-hydroxypropanoate. Yield = 44%
¹H-NMR (600MHz, CDCl₃): δ (ppm) mixture of isomers 1.01 (s, 3H), 1.02 (s, 3H), 1.18 (d, 1H, *J* 6.5 Hz), 1.20 (s, 6H), 1.45 (d, 3H, *J* 6.9 Hz), 1.66 (t, 2H, *J* 7.4 Hz), 2.32 (t, 2H, *J* 6.7 Hz), 3.98 (d, 1H, *J* 11 Hz), 4.04 (d, 1H, *J* 11 Hz), 4.09 (d, 1H, *J* 11 Hz), 4.15 (d, 1H, *J* 11 Hz), 4.22 (bq, 1H,), 4.31 (m, 1H), 5.30 (s, 1H)
¹³C-NMR (150MHz, CDCl₃): δ (ppm), mixture of isomers, 175.64, 175.61, 145.19, 145.14, 135.01,134.97, 122.69, 122.66, 77.23, 77.01, 76.80, 74.31, 74.28, 71.28, 71.25, 70.98, 67.34, 67.33, 67.26, 67.24, 66.76, 66.74, 47.31, 46.70, 46.67, 44.534, 44.526, 39.19, 30.47, 30.43, 29.76, 28.31, 28.16, 23.55, 23.52, 23.46, 23.44, 23.42, 23.36, 22.33, 22.32, 22.26, 22.25, 20.41, 20.36

### j) Preparation of a composition of matter comprising 2-[1-(3,3-dimethyl-1-cyclopenten-1-yl)ethoxy]-2-methylpropyl (2S)-2-hydroxypropanoate and 2-[1-(4,4-dimethyl-1-cyclopenten-1-yl)ethoxy]-2-methylpropyl (2S)-2-hydroxypropanoate

Same as Example 3i, except D-methyl lactate was used. Yield = 45%
¹H-NMR (600MHz, CDCl₃): δ (ppm) mixture of isomers 1.01 (s, 3H), 1.02 (s, 3H), 1.18 (d, 1H, *J* 6.5 Hz), 1.20 (s, 6H), 1.45 (d, 3H, *J* 6.9 Hz), 1.66 (t, 2H, *J* 7.4 Hz), 2.32 (t, 2H, *J* 6.7 Hz), 3.98 (d, 1H, *J* 11 Hz), 4.04 (d, 1H, *J* 11 Hz), 4.09 (d, 1H, *J* 11 Hz), 4.15 (d, 1H, *J* 11 Hz), 4.22 (bq, 1H,), 4.31 (m, 1H), 5.30 (s, 1H)
¹³C-NMR (150MHz, CDCl₃): δ (ppm), mixture of isomers, 175.64, 175.61, 145.19, 145.14, 135.01,134.97, 122.69, 122.66, 77.23, 77.01, 76.80, 74.31, 74.28, 71.28, 71.25, 70.98, 67.34, 67.33, 67.26, 67.24, 66.76, 66.74, 47.31, 46.70, 46.67, 44.534, 44.526, 39.19, 30.47, 30.43, 29.76, 28.31, 28.16, 23.55, 23.52, 23.46, 23.44, 23.42, 23.36, 22.33, 22.32, 22.26, 22.25, 20.41, 20.36

### k) Preparation of a composition of matter comprising 2-[1-(3,3-dimethyl-1-cyclopenten-1-yl)ethoxy]-2-methylpropyl 2-oxopropanoate and 2-[1-(4, 4-dimethyl-1-cyclopenten-1-yl)ethoxy]-2-methylpropyl 2-oxopropanoate

25 mL 3-neck round bottomed flask equipped with a Dean-Stark trap was charged with 0.7g of 2-(1-(3,3-dimethylcyclopent-1-en-1-yl)ethoxy)-2-methylpropan-1-ol and 2-(1-(4,4-dimethylcyclopent-1-en-1-yl)ethoxy)-2-methylpropan-1-ol as prepared in example 2, 0.63g of methyl pyruvate, 0.038g of dibutyltin oxide, 10 mL of cyclohexane and heated to reflux under N₂. After 19h the reaction mixture was cooled and concentrated on the rotovap to give 1.06g of crude concentrate. The latter was chromatographed on 80g silica cartridge using 3/1 pentane/ether as the solvent to give 0.458g of a composition of matter comprising 2-[1-(3,3-dimethyl-1-cyclopenten-1-yl)ethoxy]-2-methylpropyl 2-oxopropanoate and 2-[1-(4,4-dimethyl-1-cyclopenten-1-yl)ethoxy]-2-methylpropyl 2-oxopropanoate. Following a distillation on the kuegel-rohr 0.4g of 99% pure of a composition of matter comprising 2-[1-(3,3-dimethyl-1-cyclopenten-1-yl)ethoxy]-2-methylpropyl 2-oxopropanoate and 2-[1-(4,4-dimethyl-1-cyclopenten-1-yl)ethoxy]-2-methylpropyl 2-oxopropanoate was obtained. Yield of = 62%
¹H-NMR (600MHz, CDCl₃): δ (ppm) mixture of regioisomers 1.03 (s, 3H), 1.04 (s, 3H), 1.055 (s, 3H), 1.056 (s, 3H), 1.16 (d, 3H, *J* 6.4 Hz), 1.62-1.70 (m, 2H), 1.93 (s, 3H), 2.28-2.39 (m, 2H), 3.93 (d, 1H, *J* 11 Hz), 4.02 (d, 1H, *J* 11 Hz), 4.08 (q, 1H, *J* 6.4 Hz), 5.30 (s, 1H)
¹³C-NMR (150MHz, CDCl₃): δ (ppm), mixture of regioisomers, 190.91, 161.11, 146.05, 134.94, 128.45, 128.32, 128.20, 128.11, 128.03, 127.95, 127.87, 122.63, 74.14, 74.11, 71.85, 71.79, 67.55, 67.45, 47.60, 46.70, 44.67, 39.52, 30.64, 29.87, 29.81, 28.47, 28.33, 26.18, 26.16, 23.53, 23.43, 23.31, 23.24, 22.64, 22.58

### l) Preparation of a composition of matter comprising 2-[1-(3,3-dimethyl-1-cyclopenten-1-yl)ethoxy]-2-methylpropyl ethyl oxalate and 2-[1-(4,4-dimethyl-1-cyclopenten-1-yl)ethoxy]-2-methylpropyl ethyl oxalate

25 mL 3-neck round bottomed flask equipped with a mini Dean-Stark trap was charged with 0.7g of 2-(1-(3,3-dimethylcyclopent-1-en-1-yl)ethoxy)-2-methylpropan-1-ol and 2-(1-(4,4-dimethylcyclopent-1-en-1-yl)ethoxy)-2-methylpropan-1-ol as prepared in example 2, 0.9g of ethyl oxalate, 0.037g of dibutyltin oxide, 10 mL of cyclohexane and heated to reflux under N₂ for 14.5h then cooled to room temperature. After solvent removal, 1.47g of crude was obtained. The latter was chromatographed on silica using 5/1 pentane/ether as the solvent to afford 0.77g of a mixture containing 10% ethyloxalate and 89% of a composition of matter comprising 2-[1-(3,3-dimethyl-1-cyclopenten-1-yl)ethoxy]-2-methylpropyl ethyl oxalate and 2-[1-(4,4-dimethyl-1-cyclopenten-1-yl)ethoxy]-2-methylpropyl ethyl oxalate. The latter was distilled on the kuegel-rohr to give 0.56g of distillate containing 99% of a composition of matter comprising 2-[1-(3,3-dimethyl-1-cyclopenten-1-yl)ethoxy]-2-methylpropyl ethyl oxalate and 2-[1-(4,4-dimethyl-1-cyclopenten-1-yl)ethoxy]-2-methylpropyl ethyl oxalate. Yield = 58%
¹H-NMR (600MHz, CDCl₃): δ (ppm) mixture of regioisomers 1.00 (s, 3H), 1.01 (s, 3H), 1.19 (d, 3H, J7.8 Hz), 1.25 (s, 6H), 1.38 (t, 3H, *J* 8.6 Hz), 1.65 (t, 2H, J8.5 Hz), 2.32 (bq, 2H), 4.11 (d, 1H, *J* 13.9 Hz), 4.18 (d, 1H, *J* 13.9 Hz), 4.26 (q, 1H, *J* 7.7 Hz), 4.36 (q, 2H, *J* 8.6 Hz), 5.30 (s, 1H)
¹³C-NMR (150MHz, CDCl₃): δ (ppm), mixture of regioisomers, 157.88, 157.73, 145.20, 134.94, 77.27, 77.02, 76.76, 74.15, 72.33, 67.42, 63.03, 44.52, 39.20, 30.36, 28.29, 28.14, 23.63, 23.31, 22.34, 13.94

### m) Preparation of a composition of matter comprising 2-[1-(3,3-dimethyl-1-cyclopenten-1-yl)ethoxy]-2-methylpropyl cyclopropanecarboxylate and 2-[1-(4,4-dimethyl-1-cyclopenten-1-yl)ethoxy]-2-methylpropyl cyclopropanecarboxylate

3-neck 50 mL round bottomed flask equipped with a magnetic stir bar and a Dean-Stark trap was charged with 0.76g of 2-(1-(3,3-dimethylcyclopent-1-en-1-yl)ethoxy)-2-methylpropan-1-ol and 2-(1-(4,4-dimethylcyclopent-1-en-1-yl)ethoxy)-2-methylpropan-1-ol as prepared in example 2, 1 mL of methyl cyclopropanecarboxylate, 100 mg of dibutyltin oxide, 20 mL of cyclohexane and heated to reflux under N₂ for 18h. The reaction mixture was cooled to room temperature and concentrated on the rotovap to give 1.5g of crude. The latter was chromatographed on silica using 24/1 pentane/ether as the solvent to afford 0.47g of a composition of matter comprising 2-[1-(3,3-dimethyl-1-cyclopenten-1-yl)ethoxy]-2-methylpropyl cyclopropanecarboxylate and 2-[1-(4,4-dimethyl-1-cyclopenten-1-yl)ethoxy]-2-methylpropyl cyclopropanecarboxylate. Yield = 37%
¹H-NMR (600MHz, CDCl₃): δ (ppm) mixture of regioisomers 0.86 (m, 4H), 1.01 (s, 3H), 1.02 (s, 3H), 1.07 (d, 1H, J7.8 Hz), 1.19 (s, 3H), 1.19 (s, 3H), 1.20 (s, 3H), 1.62-1.68 (m, 3H), 2.33 (t, 2H, J7.2 Hz), 3.92 (d, 1H, *J* 11 Hz), 4.00 (d, 1H, *J* 11 Hz), 4.23 (q, 1H, *J* 6.4 Hz), 5.31 (s, 1H)
¹³C-NMR (150MHz, CDCl₃): δ (ppm), mixture of regioisomers, 174.74, 145.30, 134.82, 122.52, 77.23, 77.02, 76.81, 74.45, 69.96, 67.24, 67.15, 47.34, 46.73, 44.52, 39.21, 30.51, 29.78, 28.35, 28.19, 23.69, 23.67, 23.65, 22.33, 12.97, 8.38

### n) Preparation of a composition of matter comprising 1-(3,3-dimethyl-1-cyclopenten-1-yl)ethyl ethyl malonate and 1-(4,4-dimethyl-1-cyclopenten-1-yl)ethyl ethyl malonate

25 mL Schlenk tube equipped with a Dean-Stark trap was charged with 0.5g of a mixture comprising 1-(3,3-dimethylcyclopent-1-en-1-yl)ethan-1-ol and 1-(4,4-dimethylcyclopent-1-en-1-yl)ethan-1-ol as prepared in Example 1, 1.6g of diethylmalonate, 0.042g of dibutyltin oxide, 5 mL of cyclohexane and heated to reflux under N₂ for 2.5h, then cooled and concentrated on the rotovap. After distilling the crude concentrate on the kuegel-rohr to give 96% 0.52g of a composition of matter comprising 1-(3,3-dimethyl-1-cyclopenten-1-yl)ethyl ethyl malonate and 1-(4,4-dimethyl-1-cyclopenten-1-yl)ethyl ethyl malonate. Yield = 57%
¹H-NMR (600MHz, CDCl₃): δ (ppm) mixture of regioisomers 1.035 (s, 3H), 1.038 (s, 3H), 1.07 (d, 1H, *J* 4 Hz), 1.28 (t, 3H, J7.1 Hz), 1.35 (d, 3H, *J* 6.5 Hz), 1.69 (t, 1H, J7.2 Hz), 2.27-2.39 (m, 1H), 3.36 (s, 2H), 4.17-4.24 (m, 2H), 5.41 (s, 1H), 5.49 (q, 1H, *J* 6.5 Hz)
¹³C-NMR (150MHz, CDCl₃): δ (ppm), mixture of regioisomers, 166.65, 166.64, 165.95, 141.74, 140.26, 137.26, 125.47, 77.25, 77.03, 76.82, 70.85, 70.67, 61.46, 47.31, 46.67, 44.80, 41.98, 41.95, 38.98, 38.52, 30.80, 29.69, 29.65, 28.22, 18.91, 18.80, 14.08

### o) Preparation of a composition of matter comprising 2-[1-(3,3-dimethyl-1-cyclopenten-1-yl)ethoxy]-2-methylpropyl propiolate and 2-[1-(4,4-dimethyl-1-cyclopenten-1-yl)ethoxy]-2-methylpropyl propiolate

50 mL round bottomed flask equipped with a Dean-Stark trap was charged with 2g of 2-(1-(3,3-dimethylcyclopent-1-en-1-yl)ethoxy)-2-methylpropan-1-ol and 2-(1-(4,4-dimethylcyclopent-1-en-1-yl)ethoxy)-2-methylpropan-1-ol as prepared in example 2, 1.6g of methyl propiolate , 0.12g of dibutyltin oxide, 25 mL of cyclohexane and heated to reflux under N₂ for 48h then cooled to room temperature. After solvent removal, the crude was chromatographed on silica using 95/5 pentane/ether as the solvent to afford 1.6g of a composition of matter comprising 2-[1-(3,3-dimethyl-1-cyclopenten-1-yl)ethoxy]-2-methylpropyl propiolate and 2-[1-(4,4-dimethyl-1-cyclopenten-1-yl)ethoxy]-2-methylpropyl propiolate. The latter was distilled on the kuegel-rohr to give 1.56g of distillate containing 99% of a composition of matter comprising 2-[1-(3,3-dimethyl-1-cyclopenten-1-yl)ethoxy]-2-methylpropyl propiolate and 2-[1-(4,4-dimethyl-1-cyclopenten-1-yl)ethoxy]-2-methylpropyl propiolate. Yield = 62%
¹H-NMR (600MHz, CDCl₃): δ (ppm) mixture of regioisomers 1.01 (s, 3H), 1.02 (s, 3H), 1.19 (d, 3H, *J* 7.8 Hz), 1.23 (s, 6H), 1.67 (t, 2H, *J* 7.2 Hz), 2.33 (t, 2H, *J* 7.1 Hz), 2.89 (s, 1H), 4.03 (d, 1H, *J* 11 Hz), 4.09 (d, 1H, *J* 11 Hz), 4.23 (q, 1H, *J* 6.5 Hz), 5.31 (s, 1H)
¹³C-NMR (150MHz, CDCl₃): δ (ppm), mixture of regioisomers, 152.68, 145.16, 134.96, 77.23, 77.02, 76.81, 74.79, 74.68, 74.09, 71.60, 67.35, 44.52, 39.19, 30.38, 28.31, 28.16, 23.72, 23.36, 22.35

### p) Preparation of a composition of matter comprising 2-[1-(3,3-dimethyl-1-cyclopenten-1-yl)ethoxy]-2-methylpropyl 2-butynoate and 2-[1-(4,4-dimethyl-1-cyclopenten-1-yl)ethoxy]-2-methypropyl 2-butynoate

50 mL round bottomed flask equipped with a Dean-Stark trap was charged with 2g of 2-(1-(3,3-dimethylcyclopent-1-en-1-yl)ethoxy)-2-methylpropan-1-ol and 2-(1-(4,4-dimethylcyclopent-1-en-1-yl)ethoxy)-2-methylpropan-1-ol as prepared in example 2, 1.85g of methyl but-2-ynoate, 0.12g of dibutyltin oxide, 25 mL of cyclohexane, heated to reflux under N₂ for 24h then cooled to room temperature. After solvent removal, 2.76g of crude was obtained and chromatographed on silica using 95/5 pentane/ether as the solvent to afford 1.59g of a composition of matter comprising 2-[1-(3,3-dimethyl-1-cyclopenten-1-yl)ethoxy]-2-methylpropyl 2-butynoate and 2-[1-(4,4-dimethyl-1-cyclopenten-1-yl)ethoxy]-2-methylpropyl 2-butynoate. The latter was distilled on the kuegel-rohr to give 1.55g of distillate containing 99% of a composition of matter comprising 2-[1-(3,3-dimethyl-1-cyclopenten-1-yl)ethoxy]-2-methylpropyl 2-butynoate and 2-[1-(4,4-dimethyl-1-cyclopenten-1-yl)ethoxy]-2-methylpropyl 2-butynoate. Yield = 58%
¹H-NMR (600MHz, CDCl₃): δ (ppm) mixture of regioisomers 1.01 (s, 3H), 1.02 (s, 3H), 1.19 (d, 3H, *J* 7.8 Hz), 1.21 (s, 3H), 1.22 (s, 3H), 1.66 (t, 2H, *J* 8.6 Hz), 1.99 (s, 1H), 2.33 (t, 2H, *J* 8.5 Hz), 3.99 (d, 1H, *J* 13 Hz), 4.07 (d, 1H, *J* 13 Hz), 4.23 (q, 1H, *J* 7.7 Hz), 5.31 (s, 1H)
¹³C-NMR (150MHz, CDCl₃): δ (ppm), mixture of regioisomers, 153.79, 145.23, 134.89, 85.65, 77.28, 77.02, 76.77, 74.19, 72.45, 71.07, 67.29, 44.51, 39.12, 30.43, 28.32, 28.16, 23.74, 23.58, 23.56, 22.35, 3.82

### q) Preparation of a composition of matter comprising 2-[1-(3,3-dimethyl-1-cyclopenten-1-yl)ethoxy]-2-methylpropyl acrylate and 2-[1-(4,4-dimethyl-1-cyclopenten-1-yl)ethoxy]-2-methylpropyl acrylate

50 mL round bottomed flask equipped with a Dean-Stark trap was charged with 1g of 2-(1-(3,3-dimethylcyclopent-1-en-1-yl)ethoxy)-2-methylpropan-1-ol and 2-(1-(4,4-dimethylcyclopent-1-en-1-yl)ethoxy)-2-methylpropan-1-ol as prepared in example 2, 0.94g of ethyl acrylate, 0.059g of dibutyltin oxide, 15 mL of cyclohexane, heated to reflux under N₂ for 28h then cooled to room temperature. After solvent removal, the crude was chromatographed on silica using 95/5 pentane/ether as the solvent to afford 1g of a composition of matter comprising 2-[1-(3,3-dimethyl-1-cyclopenten-1-yl)ethoxy]-2-methylpropyl acrylate and 2-[1-(4,4-dimethyl-1-cyclopenten-1-yl)ethoxy]-2-methylpropyl acrylate. The latter was distilled on the kuegel-rohr to give 0.92g of distillate containing 99% of a composition of matter comprising 2-[1-(3,3-dimethyl-1-cyclopenten-1-yl)ethoxy]-2-methylpropyl acrylate and 2-[1-(4,4-dimethyl-1-cyclopenten-1-yl)ethoxy]-2-methylpropyl acrylate. Yield = 73%
¹H-NMR (600MHz, CDCl₃): δ (ppm) mixture of regioisomers 1.00 (s, 3H), 1.01 (s, 3H), 1.19 (d, 3H, *J* 6.5 Hz), 1.219 (s, 3H), 1.22 (s, 3H), 1.65 (t, 2H, *J* 7.1 Hz), 2.32 (t, 2H, *J* 7 Hz), 4.01 (d, 1H, *J* 11 Hz), 4.08 (d, 1H, *J* 11 Hz), 4.23 (q, 1H, *J 6.5* Hz), 5.31 (s, 1H), 5.84 (dd, 1H, *J₁* 1.3 Hz, *J₂* 10.5 Hz), 6.15 (dd, 1H, *J₁* 10.5 Hz, *J₂* 17.5 Hz), 6.42 (dd, 1H, *J₁* 1.3 Hz, *J₂* 10.5 Hz)
¹³C-NMR (150MHz, CDCl₃): δ (ppm), mixture of regioisomers, 166.05, 145.24, 134.84, 130.72, 128.52, 122.56, 77.23, 77.02, 76.81, 74.43, 70.05, 70.03, 67.28, 67.20, 47.32, 46.71, 44.52, 39.19, 30.48, 29.77, 28.34, 28.17, 23.74, 23.67, 23.63, 22.34, 22.24

### r) Preparation of a composition of matter comprising 2-[1-(3,3-dimethyl-1-cyclopenten-1-yl)ethoxy]-2-methylpropyl butyrate and 2-[1-(4,4-dimethyl-1-cyclopenten-1-yl)ethoxy]-2-methylpropyl butyrate

Prepared following conditions as reported in Example 3 f) starting with ethyl butyrate and a mixture comprising 2-(1-(3,3-dimethylcyclopent-1-en-1-yl)ethoxy)-2-methylpropan-1-ol and 2-(1-(4,4-dimethylcyclopent-1-en-1-yl)ethoxy)-2-methylpropan-1-ol as prepared in example 2.
¹H-NMR (600MHz, CDCl₃): δ (ppm) mixture of regioisomers 0.96 (t, 3H, J7.4 Hz), 1.01 (s, 3H), 1.02 (s, 3H), 1.189 (d, 3H, *J* 6.5 Hz), 1.19 (s, 3H), 1.20 (s, 3H), 1.64-1.71 (m, 4H), 2.32 (t, 2H, *J* 7.4 Hz), 3.93 (d, 1H, *J* 11 Hz), 4.01 (d, 1H, *J* 11 Hz), 4.23 (q, 1H, *J* 6.4 Hz), 5.31 (s, 1H)
¹³C-NMR (150MHz, CDCl₃): δ (ppm), mixture of regioisomers, 173.53, 145.31, 134.81, 77.25, 77.04, 76.82, 74.42, 69.87, 67.14, 44.52, 39.22, 36.30, 30.52, 28.35, 28.18, 23.69, 23.59, 22.32, 18.45, 13.74

### Example 4

### Preparation of a perfuming composition

A perfuming composition for fine fragrance was prepared by admixing the following ingredients:

| **Ingredient name** | **Parts** |
|---|---|
| 10% * benzyl acetate | 40 |
| 10% * 1,1-dimethyl-2-phenylethyl acetate | 40 |
| 10% * (Z)-3-hexenyl acetate | 20 |
| 10% * 3,7-dimethyl-6-octenyl acetate | 20 |
| 10% * (E)-3,7-dimethyl-2,6-octadienyl acetate | 20 |
| 1,5-dimethyl-1-vinyl-4-hexenyl acetate | 200 |
| 10% * ethyl 3-oxobutanoate and (2Z)-ethyl 3-hydroxy-2-butenoate | 20 |
| (2E)-2-benzylideneoctanal | 40 |
| (-)-(3aR,5aS,9aS,9bR)-3a,6,6,9a-tetramethyldodecahydronaphtho[2,1-b]furan | 40 |
| 1-((2RS,3RS)-2,3,8,8-tetramethyl-1,2,3,4,5,6,7,8-octahydronaphthalen-2-yl)ethanone, 1-[(2RS,3RS,8aRS)-2,3,8,8-tetramethyl-1,2,3,5,6,7,8,8a-octahydro-2-naphthalenyl]ethanone, 1-[(2RS,3RS,8aRS)-2,3,8,8-tetramethyl-1,2,3,4,6,7,8,8a-octahydro-2-naphthalenyl]ethanone | 3200 |
| 1% * methyl 2-aminobenzoate | 20 |
| 10% * ethyl 2-methylpentanoate | 20 |
| (Ethoxymethoxy)cyclododecane | 100 |
| Cedarwood oil | 300 |
| 10% * (3Z)-3-hexen-1-ol | 40 |
| 10% * (3Z)-hex-3-en-1-yl methyl carbonate | 80 |
| 3,7-dimethyl-6-octen-1-ol | 100 |
| 10% * (2E)-1-(2,6,6-trimethyl-1,3-cyclohexadien-1-yl)-2-buten-1-one | 10 |
| 10% * (2E)-1-(2,6,6-trimethyl-2-cyclohexen-1-yl)-2-buten-1-one | 40 |
| (2E)-2-ethyl-4-[(1R)-2,2,3-trimethyl-3-cyclopenten-1-yl]-2-buten-1-ol | 60 |
| 3-methyl-5-(2,2,3-trimethyl-3-cyclopenten-1-yl)-2-pentanol | 20 |
| 3,7-dimethyl-1,6-nonadien-3-ol | 400 |
| 10% * 3-(2/4-ethylphenyl)-2,2-dimethylpropanal | 20 |
| 10% * 3-(3-isopropyl-1-phenyl)butanal | 20 |
| 10% * 5-heptyldihydro-2(3H)-furanone | 60 |
| 10% * methyl 2-octynoate | 10 |
| Methyl 2-((1RS,2RS)-3-oxo-2-pentylcyclopentyl)acetate | 2000 |
| 3-(1,3-benzodioxol-5-yl)-2-methylpropanal | 60 |
| 1% * allyl heptanoate | 40 |
| 7-hydroxy-3,7-dimethyloctanal | 40 |
| 1% * indole | 40 |
| 10% * (Z)-3-hexenyl isobutyrate | 80 |
| (+)-Limonene | 20 |
| 3,7-dimethyl-1,6-octadien-3-ol | 200 |
| (E)-3-methyl-4-(2,6,6-trimethyl-2-cyclohexen-1-yl)-3-buten-2-one | 200 |
| (4E)-3-methyl-4-cyclopentadecen-1-one and 3-methyl-5-cyclopentadecen-1-one | 200 |
| 2-methyl-4-[(1R)-2,2,3-trimethyl-3-cyclopenten-1-yl]-4-pentenal | 10 |
| 1% * 1-(5,5-dimethyl-1-cyclohexen-1-yl)-4-penten-1-one | 20 |
| 1% * (2E,6Z)-2,6-nonadien-1-ol | 40 |
| (3Z)-3-hexen-1-yl salicylate | 40 |
| (1RS,2RS)-2,4-dimethyl-3-cyclohexene-1-carbaldehyde and (1RS,2SR)-2,4-dimethyl-3-cyclohexene-1-carbaldehyde | 10 |
| 10% * 4-hydroxy-3-methoxybenzaldehyde | 40 |
| (1RS,2RS)-2-(2-methyl-2-propanyl)cyclohexyl acetate and (1RS,2SR)-2-(2-methyl-2-propanyl)cyclohexyl acetate | 10 |
| 5,5,9,13-tetramethyl-14,16-dioxatetracyclo[11.2.1.0^{1,10}.0^{4,9} ]hexadecane | 10 |
| | **8000** |

| | |
|---|---|
| * in dipropyleneglycol | |

The addition of 2000 parts by weight of 2-[1-(3,3-dimethyl-1-cyclopenten-1-yl)ethoxy]-2-methylpropyl propionate prepared in Example 3 b) to the above-described composition imparted to the latter a musk character while bringing fruity-raspberry connotation. The musky character of the composition was perceived after 24 hours. The obtained composition corresponds to the invention's composition used in above Examples.

The addition of the same amount of 2-[1-(3,3-dimethylcyclopentyl)ethoxy]-2-methylpropyl propionate prepared in Example 3 c) imparted a dusty character allied with a slight musky connotation lower than with the invention's compound 2-[1-(3,3-dimethyl-1-cyclopenten-1-yl)ethoxy]-2-methylpropyl propionate. After 24 hours, only the dusty charactere is still perceived.

### Example 5

### Preparation of a eau de toilette comprising the invention's composition of matter

The eau de toilette was prepared by adding 15% by weight, relative to the total weight of the eau de toilette, of the invention's composition of Example 4 into ethanol.

### Example 6

### Preparation of a liquid detergent comprising the invention's compound

**Table 1: Composition of the liquid detergent formulation**

| **Ingredients** | **Concentration [wt%]** |
|---|---|
| Sodium C14-17 Alkyl Sec Sulfonate¹⁾ | 7 |
| Fatty acids, C12-18 and C18-unsaturated²⁾ | 7.5 |
| C12/14 fatty alcohol polyglycol ether with 7 mol EO³⁾ | 17 |
| Triethanolamine | 7.5 |
| Propylene Glycol | 11 |
| Citric acid | 6.5 |
| Potassium Hydroxyde | 9.5 |
| Properase L⁴⁾ | 0.2 |
| Puradax EG L⁴⁾ | 0.2 |
| Purastar ST L⁴⁾ | 0.2 |
| Acrylates/Steareth-20 Methacrylate structuring Crosspolymer⁵⁾ | 6 |
| Deionized Water | 27.4 |

| | |
|---|---|
| 1) Hostapur SAS 60; Origin: Clariant 2) Edenor K 12-18; Origin: Cognis 3) Genapol LA 070; Origin: Clariant 4) Origin: Genencor International 5) Aculyn 88; Origin: Dow Chemical | |

The liquid detergent is prepared by adding 0.5 to 1.5 % by weight, relative to the total weight of the liquid detergent, of the invention's composition of Example 4 into the unperfumed liquid detergent formulation of Table 1 under gentle shaking.

### Example 7

### Preparation of a fabric softener comprising the invention's compound

**Table 2: Composition of the softener formulation**

| **Ingredient** | **Concentration [wt%]** |
|---|---|
| Methyl bis[ethyl (tallowate)]-2- hydroxyethyl ammonium methyl sulfate¹⁾ | 12.20 |
| 1,2-benzisothiazolin-3-one²⁾ | 0.04 |
| CaCl₂ (10% aqueous solution) | 0.40 |
| Water | 87.36 |

| | |
|---|---|
| 1) Stepantex VL90 A Diester Quat; Origin: Stepan 2) Proxel GXL; Origin: Arch | |

The softener is prepared by weighting Methyl bis[ethyl (tallowate)]-2- hydroxyethyl ammonium methyl sulfate which was heated at 65°C. Then Water and 1,2-benzisothiazolin-3-one are placed in the reactor and are heated at 65°C under stirring. To the above mixture is added Methyl bis[ethyl (tallowate)]-2- hydroxyethyl ammonium methyl sulfate. The mixture is stirred 15 minutes and CaCl₂ is added. Then 0.5 to 2% by weight, relative to the total weight of the softener, of the invention's composition of Example 4 is added. The mixture is stirred 15 minutes and is cooled down to room temperature under stirring (viscosity measure : result 35 +/- 5 mPas. (shear rate 106 sec-1)).

### Example 8

### Preparation of a transparent isotropic shampoo comprising the invention's composition

**Table 3: Composition of the transparent isotropic shampoo formulation**

| **Phases** | **Ingredients** | **Concentration [wt%]** |
|---|---|---|
| **A** | Water deionized | 44.4 |
| | Polyquaternium-10 ¹⁾ | 0.3 |
| | Glycerin 85% ²⁾ | 1 |
| | DMDM Hydantoin ³⁾ | 0.2 |
| **B** | Sodium Laureth Sulfate ⁴⁾ | 28 |
| | Cocamidopropyl Betaine ⁵⁾ | 3.2 |
| | Disodium Cocoamphodiacetate ⁶⁾ | 4 |
| | Ethoxy (20) Stearyl Alcohol ⁶⁾ | 1 |
| **C** | Sodium Laureth Sulfate ⁴⁾ | 3 |
| | Glyceryl Laureate ⁷⁾ | 0.2 |
| **D** | Water deionized | 1 |
| | Sodium Methylparaben ⁸⁾ | 0.1 |
| **E** | Sodium Chloride 10% aqueous sol. | 15 |
| | Citric acid 10% aqueous sol. till pH 5.5-6 | q.s. |

| | | |
|---|---|---|
| 1) Ucare Polymer JR-400, Origin: Noveon 2) Origin: Schweizerhall 3) Glydant, Origin: Lonza 4) Texapon NSO IS, Origin: Cognis 5) Tego Betain F 50, Origin: Evonik 6) Amphotensid GB 2009, Origin: Zschimmer & Schwarz 7) Monomuls 90 L-12, Origin: Gruenau 8) Nipagin Monosodium, Origin: NIPA | | |

The shampoo is prepared by dispersed in water Polyquaternium-10. The remaining ingredients of phase A are mixed separately by addition of one after the other while mixing well after each adjunction. This pre-mix is added to the Polyquaternium-10 dispersion and mixed for another 5 min. Then, the premixed phase B and the premixed Phase C are added (Monomuls 90L-12 is heated to melt in Texapon NSO IS) while agitating. Phase D and Phase E are added while agitating. PH is adjusted with citric acid solution till pH: 5.5 - 6.0 leading to an unperfumed shampoo formulae.

The perfumed shampoo is prepared by adding 0.4 to 0.8% by weight, relative to the total weight of the shampoo, of the invention's composition of Example 4 into the unperfumed shampoo formulation of Table 3 under gentle shaking.

### Example 9

### Preparation of a structured shower gel comprising the invention's composition

**Table 4: Composition of the shower gel formulation**

| **Ingredients** | **Amount (% wt)** |
|---|---|
| WATER deionised | 49.350 |
| Tetrasodium EDTA ¹⁾ | 0.050 |
| Acrylates Copolymer²⁾ | 6.000 |
| Sodium C12-C15 Pareth Sulfate ³⁾ | 35.000 |
| Sodium Hydroxide 20% aqueous solution | 1.000 |
| Cocamidopropyl Betaine⁴⁾ | 8.000 |
| Methylchloroisothiazolinone and Methylisothiazolinone⁵⁾ | 0.100 |
| Citric Acid (40%) | 0.500 |

| | |
|---|---|
| 1) EDETA B POWDER; trademark and origin: BASF 2) CARBOPOL AQUA SF-1 POLYMER; trademark and origin: NOVEON 3) ZETESOL AO 328 U; trademark and origin: ZSCHIMMER & SCHWARZ 4) TEGO-BETAIN F 50; trademark and origin: GOLDSCHMIDT 5) KATHON CG; trademark and origin: ROHM & HASS | |

The shower gel is prepared by adding 0.5 to 1.5% by weight, relative to the total weight of the shower gel, of the invention's composition of Example 4 into the unperfumed shower gel formulation of Table 4 under gentle shaking.

### Example 10

### Preparation of a transparent shower gel comprising the invention's composition

**Table 5: Composition of the transparent shower gel formulation**

| **Ingredients** | **Concentration (% wt)** |
|---|---|
| WATER deionized | 52.40 |
| Tetrasodium EDTA ¹⁾ | 0.10 |
| Sodium Benzoate | 0.50 |
| Propylene Glycol | 2.00 |
| Sodium C12-C15 Pareth Sulfate ²⁾ | 35.00 |
| Cocamidopropyl Betaine³⁾ | 8.00 |
| Polyquaternium-7⁴⁾ | 0.20 |
| Citric Acid (40%) | 1.00 |
| Sodium Chloride | 0.80 |

| | |
|---|---|
| 1) EDETA B POWDER; trademark and origin: BASF 2) ZETESOL AO 328 U; trademark and origin: ZSCHIMMER & SCHWARZ 3) TEGO-BETAIN F 50; trademark and origin: GOLDSCHMIDT 4) MERQUAT 550; trademark and origin: LUBRIZOL | |

The transparent shower gel is prepared by adding 0.5 to 1.5% by weight, relative to the total weight of the shower gel, of the invention's composition of Example 4 into the unperfumed shower gel formulation of Table 5 under gentle shaking.

### Example 11

### Preparation of a milky shower gel comprising the invention's composition

**Table 6: Composition of the milky shower gel formulation**

| **Ingredients** | **Concentration (% wt)** |
|---|---|
| WATER deionized | 50.950 |
| Tetrasodium EDTA ¹⁾ | 0.050 |
| Sodium Benzoate | 0.500 |
| Glycerin 86% | 3.500 |
| Sodium Laureth Sulfate ²⁾ | 27.000 |
| Polyquaternium-7³⁾ | 1.000 |
| Coco-Betaine⁴⁾ | 6.000 |
| PEG-120 Methyl Glucose trioleate⁵⁾ | 1.000 |
| Citric Acid (40%) | 1.000 |
| Glycol Distearate & Laureth-4 & Cocamidopropyl Betaine⁶⁾ | 3.000 |
| Sodium Chloride 20% | 5.000 |
| PEG-40 Hydrogenated Castor oil⁷⁾ | 1.000 |

| | |
|---|---|
| 1) EDETA B POWDER; trademark and origin: BASF 2) Texapon NSO IS; trademark and origin: COGNIS 3) MERQUAT 550; trademark and origin: LUBRIZOL 4) DEHYTON AB-30; trademark and origin: COGNIS 5) GLUCAMATE LT; trademark and origin: LUBRIZOL 6) EUPERLAN PK 3000 AM; trademark and origin: COGNIS 7) CREMOPHOR RH 40; trademark and origin: BASF | |

The transparent shower gel is prepared by adding 0.5 to 1.5% by weight, relative to the total weight of the shower gel, of the invention's composition of Example 4 into the unperfumed shower gel formulation of Table 6 under gentle shaking.

### Example 12

### Preparation of a pearly shampoo comprising the invention's composition

**Table 7: Composition of the pearly isotropic_shampoo formulation**

| **Phases** | **Ingredients** | **Concentration (% wt)** |
|---|---|---|
| **A** | Water deionized | 45.97 |
| | Tetrasodium EDTA ¹⁾ | 0.05 |
| | Guar Hydroxypropyltrimonium Chloride ²⁾ | 0.05 |
| | Polyquaternium-10 ³⁾ | 0.075 |
| **B** | NaOH 10% aqueous sol. | 0.3 |
| **C** | Ammonium Lauryl Sulfate ⁴⁾ | 34 |
| | Ammonium Laureth Sulfate ⁵⁾ | 9.25 |
| | Cocamidopropyl Betaine ⁶⁾ | 2 |
| | Dimethicone (&) C12-13 Pareth-4 (&) C12-13 Pareth-23 (&) Salicylic Acid ⁷⁾ | 2.5 |
| **D** | Cetyl Alcohol ⁸⁾ | 1.2 |
| | Cocamide MEA ⁹⁾ | 1.5 |
| | Glycol Distearate ¹⁰⁾ | 2 |
| **E** | Methylchloroisothiazolinone & Methylisothiazolinone 11) | 0.1 |
| | D-Panthenol 75% ¹²⁾ | 0.1 |
| | Water deionized | 0.3 |
| **F** | Sodium Chloride 25% aqueous sol. | 0.6 |

| | | |
|---|---|---|
| 1) EDETA B Powder, Origin: BASF 2) Jaguar C14 S, Origin: Rhodia 3) Ucare Polymer JR-400, Origin: Noveon 4) Sulfetal LA B-E, Origin: Zschimmer & Schwarz 5) Zetesol LA, Origin: Zschimmer & Schwarz 6) Tego Betain F 50, Origin: Evonik 7) Xiameter MEM-1691, Origin: Dow Corning 8) Lanette 16, Origin: BASF 9) Comperlan 100, Origin: Cognis 10) Cutina AGS, Origin: Cognis 11) Kathon CG, Origin: Rohm & Haas 12) D-Panthenol, Origin: Roche | | |

The shampoo is prepared by dispersed in water and Tetrasodium EDTA, Guar Hydroxypropyltrimonium Chloride and Polyquaternium-10. NaOH 10% solution (Phase B) is added once Phase A is homogeneous. Then, the premixed Phase C is added. and mixture is heated to 75°C. Phase D ingredients are added and mixed till homogeneous. The mixture is cooled down. At 45°C, Phase E ingredients are added while mixing. Final viscosity is adjusted with 25% NaCl solution and pH of 5.5-6 is adjusted with 10% NaOH solution.

The perfumed pearly shampoo is prepared by adding 0.4 to 0.8% by weight, relative to the total weight of the shampoo, of the invention's composition of Example 4 into the unperfumed shampoo formulation of Table 7 under gentle shaking.

### Example 13

### Preparation of a structured shower gel comprising the invention's composition

**Table 8: Composition of the milky shower gel formulation**

| **Ingredients** | **Amount (% wt)** |
|---|---|
| WATER deionised | 49.350 |
| Tetrasodium EDTA ¹⁾ | 0.050 |
| Acrylates Copolymer²⁾ | 6.000 |
| Sodium C12-C15 Pareth Sulfate ³⁾ | 35.000 |
| Sodium Hydroxide 20% aqueous solution | 1.000 |
| Cocamidopropyl Betaine⁴⁾ | 8.000 |
| Methylchloroisothiazolinone and Methylisothiazolinone⁵⁾ | 0.100 |
| Citric Acid (40%) | 0.500 |

| | |
|---|---|
| 6) EDETA B POWDER; trademark and origin: BASF 7) CARBOPOL AQUA SF-1 POLYMER; trademark and origin: NOVEON 8) ZETESOL AO 328 U; trademark and origin: ZSCHIMMER & SCHWARZ 9) TEGO-BETAIN F 50; trademark and origin: GOLDSCHMIDT 10)KATHON CG; tradeark and origin: ROHM & HASS | |

The transparent shower gel is prepared by adding 0.5 to 1.5% by weight, relative to the total weight of the shower gel, of the invention's composition of Example 4 into the unperfumed shower gel formulation of Table 8 under gentle shaking.

### Example 14

### Preparation of anhydrous antiperspirant spray formulations comprising the invention's composition

**Table 9: Composition of the anhydrous antiperspirant spray formulation**

| **Ingredients** | **Amount (% wt)** |
|---|---|
| Cyclomethicone ⁽¹⁾ | 53.0 |
| Isopropyl myristate | 9.0 |
| Silica ⁽²⁾ | 1.0 |
| Quaternium-18-hectorite ⁽³⁾ | 3.3 |
| Aluminium chlorohydrate ⁽⁴⁾ | 32.7 |
| Perfume oil | 1 |

| | |
|---|---|
| ⁽¹⁾ Dow Corning^{®} 345 Fluid; origin: Dow Corning ⁽²⁾ Aerosil^{®} 200 ; origin: Evonik ⁽³⁾ Bentone^{®} 38; origin: Elementis Specialities ⁽⁴⁾ Micro Dry Ultrafine; origin: Reheis | |

Anhydrous antiperspirant spray formulation is prepared by using a high speed stirrer. Silica and Quaternium-18-hectorite are added to the mixture of isopropyl myristate and cyclomethicone. Once completely swollen, aluminium chlorohydrate is added portion-wise under stirring until the mixture becomes homogeneous and without lumps. Then a perfume oil being the invention's composition of Example 4 is added.

### Example 15

### Preparation of deodorant spray emulsion formulations comprising the invention's composition

**Table 10: Composition of deodorant spray emulsion formulation**

| **Ingredients** | **Amount (% wt)** |
|---|---|
| Ethanol (95%) | 89.25 |
| Triclosan ⁽¹⁾ | 0.25 |
| Isopropyl myristate | 9.00 |
| Invention's composition of Example 4 | 1.5 |

| | |
|---|---|
| ⁽¹⁾ Irgasan^{®} DP 300; origin: BASF ⁽²⁾ mixture of Compounds 2a/2b ca. 45:55 | |

Deodorant spray emulsion formulation is prepared by mixing and dissolving all the ingredients according to the sequence of Table 10. Aerosol cans are filled, and the propellant is crimped and added. Aerosol filling: 40% active solution 60% propane / butane (2.5 bar).

### Example 16

### Preparation of deodorant stick formulations comprising the invention's composition

**Table 11: Composition of Deodorant stick formulation**

| **Phase** | **Ingredients** | **Amount (% wt)** |
|---|---|---|
| A | Stearic acid | 5.00 |
| | 1,2-Propylene glycol | 41.45 |
| | Sodium hydroxide (20% aqueous solution) | 4.20 |
| | Water | 30.00 |
| | Tetrasodium EDTA ⁽¹⁾ | 0.10 |
| | Ceteareth-25 ⁽²⁾ | 1.50 |
| | PPG-3 Myristyl ether ⁽³⁾ | 1.50 |
| B | 1,2-Propylene glycol | 15.00 |
| | Triclosan ⁽⁴⁾ | 0.25 |
| C | Perfume oil | 1 |

| | | |
|---|---|---|
| ⁽¹⁾ Edeta^{®} B Power; origin: BASF ⁽²⁾ Cremophor^{®} A25; origin: BASF ⁽³⁾ Tegosoft^{®} APM; origin: Evonik ⁽⁴⁾ Irgasan^{®} DP 300; origin: BASF | | |

Deodorant stick formulation is btained by weighing all the components of Part A and heating to 70-75°C. Ceteareth-25 is added once the other Part A ingredients are mixed and heated. When the Ceteareth-25 is dissolved, stearic acid is added. Part B is prepared by dissolving Triclosan in 1,2-propylene glycol. Evaporated water is compensated. Then, slowly, under mixing, Part B is poured into Part A. A perfume oil being the invention's composition of Example 4 (Phase C) is added under gentle shaking. To stock, a plastic bag is put into the bucket to be sealed after cooling. Moulds were filled at about 70°C.

### Example 17

### Preparation of deodorant roll-on formulations comprising the invention's composition

**Table 12: Composition of deodorant roll-on formulation**

| **Phase** | **Ingredients** | **Amount (% wt)** |
|---|---|---|
| A | Water | 50.00 |
| | Hydroxyethylcellulose ⁽¹⁾ | 0.70 |
| B | Ethanol (95%) | 40.00 |
| | 1,2-Propylene glycol | 5.00 |
| | Triclosan ⁽²⁾ | 0.30 |
| C | PEG-40 hydrogenated castor oil ⁽³⁾ | 3.00 |
| D | Invention's composition of Example 4 | 1 |

| | | |
|---|---|---|
| ⁽¹⁾ Natrosol^{®} 250 H; origin: Ashland ⁽²⁾ Irgasan^{®} DP 300; origin: BASF ⁽³⁾ Cremophor^{®} RH 40; origin: BASF | | |

Part A is prepared by sprinkling little-by-little the hydroxyethylcellulose into the water, whilst rapidly stirring with a turbine until the hydroxyethylcellulose is entirely swollen giving a limpid gel. Part B is slowly poured into Part A, whilst continuing stirring until the entire mixture is homogeneous. Then Parts C and D are added under gentle shaking.

### Example 18

### Preparation of day cream base O/W emulsions comprising the invention's composition

**Table 13: Composition of day cream base O/W emulsion formulation**

| **Phase** | **Ingredients** | **Amount (% wt)** |
|---|---|---|
| A | Steareth-2 (and) PEG-8 Distearate⁽¹⁾ | 5.0 |
| | Cetyl alcohol | 0.5 |
| | Ceteth-20 (AND) glyceryl stearate (and) PEG-6 stearate (and) Steareth-20 ⁽²⁾ | 4.0 |
| | Squalan ⁽³⁾ | 1.0 |
| | Paraffin oil ⁽⁴⁾ | 2.0 |
| | Petrolatum ⁽⁵⁾ | 5.5 |
| B | Deionized water | 75.9 |
| | Propylene glycol | 5.0 |
| C | Phenoxyethanol (AND) Piroctone olamine ⁽⁶⁾ | 0.6 |
| D | Sodium carbomer ⁽⁷⁾ | 0.2 |
| E | Perfume oil | 0.3 |

| | | |
|---|---|---|
| ⁽¹⁾ Arlacel^{®} 985; origin: Croda ⁽²⁾ Tefose^{®} 2561; origin: Gattefossé ⁽³⁾ Biolip P 90; origin: Gattefossé ⁽⁴⁾ Mineral oil 30-40 CPS (30-40 mPa*s) ⁽⁵⁾ Petroleum jelly ⁽⁶⁾ Nipaguard^{®} PO 5; origin: Clariant ⁽⁷⁾ PNC 400 | | |

Day cream base O/W emulsions is prepared by heating Phases A and B separately to 70-75 °C. Phase A is added to Phase B, then vacuum is applied. The mixture is stirred and cooled to 55°C for 15 min. After cooling to room temperature, phenoxyethanol (and) piroctone olamine (Part C) are added when a temperature of 45°C is reached. The mixture is stirred for 5 min before sodium carbomer (Part D) and a perfume oil being the invention's composition of Example 4 (Part E) is added. The mixture is stirred for 3 min, then the stirring was stopped for 15 min. When the temperature of the mixture reaches 30°C, the stirring is resumed for another 15 min until the cream becomes homogeneous, glossy and without lumps. If necessary the pH is adjusted to 6.70-7.20 with Glydant, Phenonip or Nipaguard PO5 or to 6.30-7.00 with Nikkoguard.

## Claims

1. Use as perfuming ingredient of a compound of formula (I), 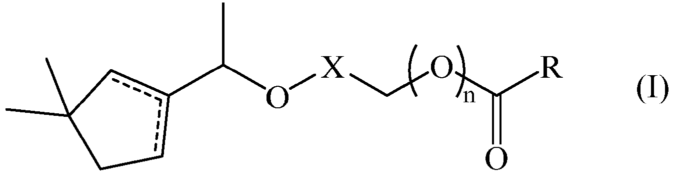 in the form of any one of its stereoisomers or as a mixture thereof, wherein one dotted line represents a carbon-carbon double bond and the other dotted line represents a carbon-carbon single bond; n is 0 or 1; X is a C(=O) or C(Me)₂ group; R is a hydrogen atom, a C₁₋₆ alkyl, C₁₋₆ alkoxyl, C₂₋₆ alkenyl, C₂₋₆ alkynyl or C₃₋₆ cycloalkyl group, a C(=O)R' group or a CH(OR")R" group wherein R' is a C₁₋₃ alkyl or C₁₋₃ alkoxyl group and R", independently from each other, is a hydrogen atom or a C₁₋₃ alkyl group.

2. The use according to claim 1, wherein when n is 0 then R is a C₁₋₆ alkoxyl group.

3. The use according to any one of claims 1 to 2, wherein n is 1.

4. The use according to any one of claims 1 to 3, wherein the compound of formula (I) is a compound of formula 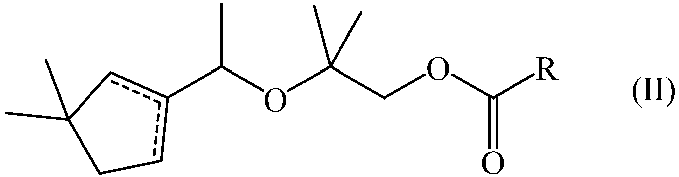 in the form of any one of its stereoisomers or as a mixture thereof, wherein the dotted lines and R have the same meaning as defined in claims 1 to 3.

5. The use according to any one of claims 1 to 4, wherein R is a C₁₋₃ alkyl, C₁₋₃ alkoxyl, C₂₋₃ alkenyl, C₂₋₃ alkynyl or C₃ cycloalkyl group or a C(=O)R' group wherein R' is a C₁₋₂ alkyl group.

6. The use according to any one of claims 1 to 5, wherein R is a ethyl, propyl, ethoxy, prop-1-en-1-yl, 2-ethynyl or a cyclopropyl group or a C(=O)Me group.

7. The use according to any one of claims 1 to 6, wherein R is an ethyl group.

8. The use according to any one of claims 1 to 7, wherein the compound of formula (I) is a compound of formula 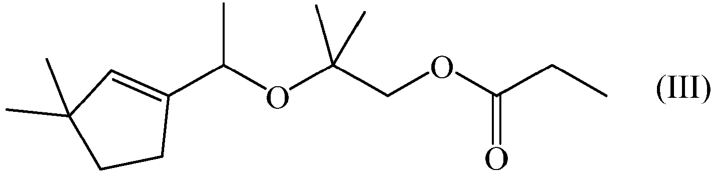 in the form of any one of its stereoisomers or as a mixture thereof.

9. A method to confer, enhance, improve or modify the odor properties of a perfuming composition or of a perfumed article or of a surface, which method comprises adding to said composition or article an effective amount of at least a compound of formula (I) as defined in claims 1 to 8.

10. A compound of formula of formula (I) as defined in claims 1 to 8.

11. A perfuming composition comprising
i) at least one compound of formula (I), as defined in claim 10;
ii) at least one ingredient selected from the group consisting of a perfumery carrier and a perfumery base; and
iii) optionally at least one perfumery adjuvant.

12. A perfumed consumer product comprising at least one compound of formula (I), as defined in claim 10 or a perfuming composition as defined in claim 11.

13. The perfumed consumer product according to claim 12, **characterized in that** the perfumery consumer product is a perfume, a fabric care product, a body-care product, a cosmetic preparation, a skin-care product, an air care product or a home care product.

14. The perfumed consumer product according to claim 13, **characterized in that** the perfumery consumer product is a fine perfume, a splash or eau de parfum, a cologne, a shave or after-shave lotion, a liquid or solid detergent optionally in the form of a pod or tablet, a fabric softener, a liquid or solid scent booster, a dryer sheet, a fabric refresher, an ironing water, a paper, a bleach, a carpet cleaner, a curtain-care product, a shampoo, a leave-on or rinse-off hair conditioner, a coloring preparation, a color-care product, a hair shaping product, a dental care product, a disinfectant, an intimate care product, a hair spray, a skin cream or lotion, a vanishing cream, a deodorant or antiperspirant, a hair remover, a tanning or sun or after sun product, a nail product, a skin cleansing, a makeup, a perfumed soap, a shower or bath mousse, oil or gel, a foot/hand care product, a hygiene product, an air freshener, a "ready to use" powdered air freshener, a mold remover, a furnisher care, a wipe, a dish detergent or hard-surface detergent, a leather care product, a car care product.

## Patentansprüche

1. Verwendung einer Verbindung der Formel (I) als parfümierenden Inhaltsstoff 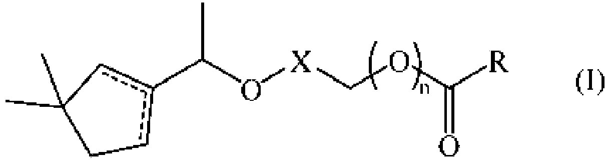 in Form eines seiner Stereoisomere oder als Gemisch davon, wobei eine gestrichelte Linie eine Kohlenstoff-Kohlenstoff-Doppelbindung darstellt und die andere gestrichelte Linie eine Kohlenstoff-Kohlenstoff-Einfachbindung darstellt; n 0 oder 1 ist; X eine C(=O)- oder C(Me)₂-Gruppe ist; R ein Wasserstoffatom, eine C₁₋₆-Alkyl-, C₁₋₆-Alkoxyl-, C₂₋₆-Alkenyl-, C₂₋₆-Alkinyl- oder C₃₋₆-Cycloalkyl-Gruppe, eine C(=O)R'-Gruppe oder eine CH(OR")R"-Gruppe ist, wobei R' eine C₁₋₃-Alkyl- oder C₁₋₃-Alkoxylgruppe ist und R", unabhängig voneinander, ein Wasserstoffatom oder eine C₁₋₃-Alkylgruppe ist.

2. Verwendung nach Anspruch 1, wobei, wenn n 0 ist, R eine C₁₋₆-Alkoxylgruppe ist.

3. Verwendung nach einem der Ansprüche 1 bis 2, wobei n 1 ist.

4. Verwendung nach einem der Ansprüche 1 bis 3, wobei die Verbindung der Formel (I) eine Verbindung der folgenden Formel ist: 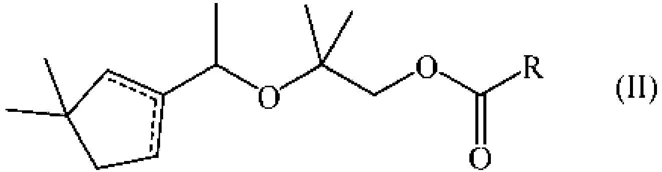 in Form eines seiner Stereoisomere oder als Gemisch davon, wobei die gestrichelten Linien und R dieselbe Bedeutung wie in den Ansprüchen 1 bis 3 definiert aufweisen.

5. Verwendung nach einem der Ansprüche 1 bis 4, wobei R eine C₁₋₃-Alkyl-, C₁₋₃-Alkoxyl-, C₂₋₃-Alkenyl-, C₂₋₃-Alkinyl- oder C₃-Cycloalkyl-Gruppe oder eine C(=O)R'-Gruppe ist, wobei R' eine C₁₋₂-Alkylgruppe ist.

6. Verwendung nach einem der Ansprüche 1 bis 5, wobei R eine Ethyl-, Propyl-, Ethoxy-, Prop-1-en-1-yl-, 2-Ethinyl- oder eine Cyclopropyl-Gruppe oder eine C(=O)Me-Gruppe ist.

7. Verwendung nach einem der Ansprüche 1 bis 6, wobei R eine Ethylgruppe ist.

8. Verwendung nach einem der Ansprüche 1 bis 7, wobei die Verbindung der Formel (I) eine Verbindung der folgenden Formel ist: 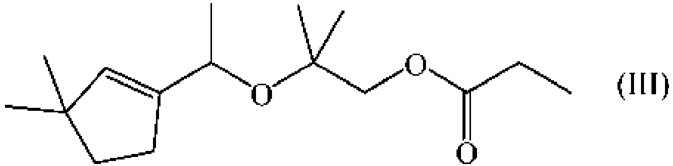 in Form eines seiner Stereoisomere oder als Gemisch davon.

9. Verfahren, um die Geruchseigenschaften einer parfümierenden Zusammensetzung oder eines parfümierten Artikels oder einer Oberfläche zu verleihen, zu verstärken, zu verbessern oder zu modifizieren, wobei das Verfahren Hinzufügen einer wirksamen Menge mindestens einer Verbindung der Formel (I), wie in den Ansprüchen 1 bis 8 definiert, zu der Zusammensetzung oder dem Artikel umfasst.

10. Verbindung der Formel (I), wie in den Ansprüchen 1 bis 8 definiert.

11. Parfümierende Zusammensetzung, umfassend
i) mindestens eine Verbindung der Formel (I), wie in Anspruch 10 definiert;
ii) mindestens einen Inhaltsstoff, ausgewählt aus der Gruppe bestehend aus einem Parfümträger und einer Parfümbasis; und
iii) gegebenenfalls mindestens einen Parfümhilfsstoff.

12. Parfümiertes Verbraucherprodukt, das mindestens eine Verbindung der Formel (I), wie in Anspruch 10 definiert, oder eine parfümierende Zusammensetzung, wie in Anspruch 11 definiert, umfasst.

13. Parfümiertes Verbraucherprodukt nach Anspruch 12, **dadurch gekennzeichnet, dass** das Parfüm-Verbraucherprodukt ein Parfüm, ein Textilpflegeprodukt, ein Körperpflegeprodukt, eine kosmetische Zubereitung, ein Hautpflegeprodukt, ein Luftpflegeprodukt oder ein Haushaltspflegeprodukt ist.

14. Parfümiertes Verbraucherprodukt nach Anspruch 13, **dadurch gekennzeichnet, dass** das Parfüm-Verbraucherprodukt ein edles Parfüm, ein Splash oder Eau de Parfum, ein Kölnischwasser, eine Rasier- oder Aftershave-Lotion, ein flüssiges oder festes Waschmittel, gegebenenfalls in Form eines Pods oder einer Tablette, ein Weichspüler, ein flüssiger oder fester Duftverstärker, ein Trocknertuch, ein Textilerfrischer, ein Bügelwasser, ein Papier, eine Bleiche, ein Teppichreiniger, ein Gardinenpflegeprodukt, ein Shampoo, eine Leave-in- oder Ausspül-Haarspülung, eine färbende Zubereitung, ein Farbpflegeprodukt, ein Haarstylingprodukt, ein Zahnpflegeprodukt, ein Desinfektionsmittel, ein Intimpflegeprodukt, ein Haarspray, eine Hautcreme oder -lotion, eine Tagescreme, ein Deodorant oder Antitranspirant, ein Enthaarungsmittel, ein Bräunungs- oder Sonnenschutz- oder After-Sun-Produkt, ein Nagelprodukt, ein Hautreiniger, ein Make-up, eine parfümierte Seife, ein Dusch- oder Badeschaum, -öl oder -gel, ein Fuß-/Handpflegeprodukt, ein Hygieneprodukt, ein Lufterfrischer, ein gebrauchsfertiger pulverförmiger Lufterfrischer, ein Schimmelentferner, ein Möbelpflegemittel, ein Reinigungstuch, ein Geschirrspülmittel oder Reinigungsmittel für harte Oberflächen, ein Lederpflegeprodukt, ein Autopflegeprodukt ist.

## Revendications

1. Utilisation comme ingrédient parfumant d'un composé de formule (I), 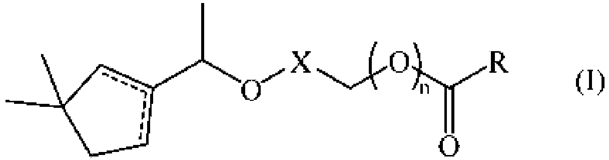 sous la forme de l'un quelconque de ses stéréoisomères ou sous forme d'un mélange de ceux-ci, dans lequel une ligne pointillée représente une liaison double carbone-carbone et l'autre ligne pointillée représente une liaison simple carbone-carbone ; n vaut 0 ou 1 ; X est un C(=O) ou un groupe C(Me)₂ ; R est un atome d'hydrogène, un groupe alkyle en C₁₋₆, un groupe alcoxyle en C₁₋₆, un groupe alcényle en C₂₋₆, un groupe alcynyle en C₂₋₆ ou un groupe cycloalkyle en C₃₋₆, un groupe C(=O)R' ou un groupe CH(OR")R" dans lequel R' est un groupe alkyle en C₁₋₃ ou un groupe alcoxyle en C₁₋₃ et R", indépendamment l'un de l'autre, est un atome d'hydrogène ou un groupe alkyle en C₁₋₃.

2. Utilisation selon la revendication 1, dans laquelle lorsque n vaut 0, R est un groupe alcoxyle en C₁₋₆.

3. Utilisation selon l'une quelconque des revendications 1 à 2, dans laquelle n vaut 1.

4. Utilisation selon l'une quelconque des revendications 1 à 3, dans laquelle le composé de formule (I) est un composé de formule 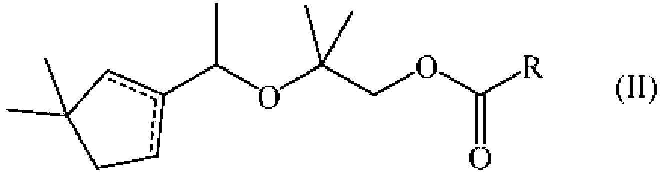 sous la forme de l'un quelconque de ses stéréoisomères ou sous forme d'un mélange de ceux-ci, dans lequel les lignes pointillées et R ont la même signification que celle définie dans les revendications 1 à 3.

5. Utilisation selon l'une quelconque des revendications 1 à 4, dans laquelle R est un groupe alkyle en C₁₋₃, un groupe alcoxyle en C₁₋₃, un groupe alcényle en C₂₋₃, un groupe alcynyle en C₂₋₃ ou un groupe cycloalkyle en C₃ ou un groupe C(=O)R' dans lequel R' est un groupe alkyle en C₁₋₂.

6. Utilisation selon l'une quelconque des revendications 1 à 5, dans laquelle R est un groupe éthyle, propyle, éthoxy, prop-1-én-1-yle, 2-éthynyle ou cyclopropyle ou un groupe C(=O)Me.

7. Utilisation selon l'une quelconque des revendications 1 à 6, dans laquelle R est un groupe éthyle.

8. Utilisation selon l'une quelconque des revendications 1 à 7, dans laquelle le composé de
formule (I) est un composé de formule 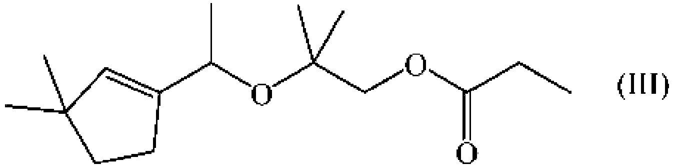 sous la forme de l'un quelconque de ses stéréoisomères ou sous forme d'un mélange de ceux-ci.

9. Procédé pour conférer, renforcer, améliorer ou modifier les propriétés odorantes d'une composition parfumante, d'un article parfumé ou d'une surface, lequel procédé comprend l'ajout à ladite composition ou audit article d'une quantité efficace d'au moins un composé de formule (I) selon les revendications 1 à 8.

10. Composé de formule de formule (I) selon les revendications 1 à 8.

11. Composition parfumante comprenant
i) au moins un composé de formule (I), selon la revendication 10 ;
ii) au moins un ingrédient choisi parmi le groupe constitué d'un support de parfumerie et d'une base de parfumerie ; et
iii) facultativement au moins un adjuvant de parfumerie.

12. Produit de consommation parfumé comprenant au moins un composé de formule (I), selon la revendication 10 ou une composition parfumante selon la revendication 11.

13. Produit de consommation parfumé selon la revendication 12, **caractérisé en ce que** le produit de consommation de parfumerie est un parfum, un produit d'entretien du linge, un produit de soin du corps, une préparation cosmétique, un produit de soin de la peau, un produit d'assainissement de l'air ou un produit d'entretien ménager.

14. Produit de consommation parfumé selon la revendication 13, **caractérisé en ce que** ce produit est un parfum, une eau de parfum, une eau de Cologne, une lotion de rasage ou d'après-rasage, un détergent liquide ou solide, éventuellement sous forme de dosette ou de tablette, un adoucissant, un rehausseur de parfum liquide ou solide, une feuille pour sèche-linge, un désodorisant de textile, une eau de repassage, du papier, de l'eau de Javel, un nettoyant pour tapis, un produit d'entretien pour rideaux, un shampooing, un après-shampooing avec ou sans rinçage, une préparation colorante, un produit de soin de la couleur, un produit de coiffage, un produit d'hygiène dentaire, un désinfectant, un produit d'hygiène intime, une laque, une crème ou lotion pour le corps, une crème de jour, un déodorant ou un antitranspirant, une crème dépilatoire, un produit autobronzant ou après-soleil, un produit pour les ongles, un nettoyant pour la peau, un maquillage, un savon parfumé, une mousse, une huile ou un gel de douche ou de bain, un produit de soin pour les pieds/mains, un produit d'hygiène, un désodorisant, un désodorisant en poudre « prêt à l'emploi », un produit anti-moisissures, un produit d'entretien des meubles, une lingette, un détergent de vaisselle ou un détergent pour surfaces dures, un produit d'entretien du cuir, un produit d'entretien automobile.
